# EUROPEAN PATENT APPLICATION

(11) **EP 4 840 849 A1**
(43) Date of publication of application: **18.11.2026**
(21) Application number: 25738518.7
(22) Date of filing: 09.01.2025
(51) Int. Cl.: C07D 215/14, C07D 221/04, C07D 221/06, A61P 17/00, A61P 17/06, A61P 17/10, A61K 31/192

(54) **BIPHENYL COMPOUND, PREPARATION METHOD THEREFOR, AND INTERMEDIATE, PHARMACEUTICAL COMPOSITION AND USE THEREOF**

(30) Priority: 09.01.2024 CN 202410033728; 26.12.2024 CN 202411943863
(71) Applicant: Jiangxi Kvvit Pharmaceutical Co., Ltd., Ganzhou, Jiangxi 341001 (CN)
(72) Inventor: YAN, Shousheng, Ganzhou, Jiangxi 341001 (CN); HE, Honghong, Ganzhou, Jiangxi 341001 (CN); GUAN, Xitao, Ganzhou, Jiangxi 341001 (CN); CHEN, Shuoji, Ganzhou, Jiangxi 341001 (CN)
(74) Representative: Mewburn Ellis LLP
(86) International application number: PCT/CN2025/071558
(87) International publication number: WO 2025/148990

(57) **Abstract**

Disclosed in the present invention are a biphenyl compound, a preparation method therefor, and an intermediate, a pharmaceutical composition and the use thereof. Specifically provided is a compound as shown in formula I or a pharmaceutically acceptable salt thereof. The compound has good activity and selectivity on a retinoic acid receptor γ agonist, almost has no agonistic activity on retinoic acid receptors α and/or β, and is thus beneficial to reducing systemic side effects. Preferably, the compound has moderately partial agonistic activity, has low skin irritation and few adverse reactions while maintaining its efficacy, and thus has good safety and compliance, such that the problems generally present in existing RAR compounds are effectively solved, and the compound can be used for treating and preventing diseases related to retinoic acid receptor γ.

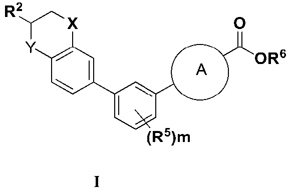

## Description

The present application claims priority to Chinese Patent Application 2024100337288 filed on January 09, 2024 and Chinese Patent Application 2024119438632 filed on December 26, 2024. The contents of the above Chinese patent applications are incorporated herein by reference in their entirety.

### TECHNICAL FIELD

The present disclosure relates to a biphenyl compound, a preparation method therefor, an intermediate thereof, a pharmaceutical composition thereof, and a use thereof.

### BACKGROUND

Retinoic acid receptor (RAR) is a ligand-activated transcription factor belonging to the nuclear receptor superfamily, and its main ligands are retinoids. After binding to its ligand retinoic acid, RAR can form a heterodimer with RXR (Retinoid X receptor) and bind to specific genes, thereby controlling the expression of specific genes, playing an important role in processes such as cell growth, differentiation, and apoptosis, and participating in physiological and pathological processes such as embryonic development, tissue differentiation, and tumors. RAR includes three subtypes: RARα, RARβ, and RARγ, wherein RARα is widely distributed in most tissues such as lung, spleen, and gallbladder, RARβ is mainly expressed in placenta, kidney, heart, nervous system, etc., and RARγ is mainly expressed in skin, epithelium, and cartilage tissues.

Synthetic or endogenous retinoic acid (vitamin A acid) substances can regulate gene transcription and expression in suprabasal cells by binding to and activating RARγ in the skin tissue, thereby indirectly regulating keratinization, promoting normal keratinization of the epidermis, and inhibiting excessive proliferation of keratinocytes. Clinically available retinoids such as tretinoin, isotretinoin, acitretin, etretinate, aromatic retinoic acid, ethyl ester of aromatic retinoic acid, adapalene, and tazarotene are used in the treatment of psoriasis, skin keratinization diseases, eczema, acne, etc. However, since these retinoid drugs are non-selective RAR drugs, they can bind to receptors such as RARα, RARβ, RARγ, and RXR, and while exerting therapeutic effects in promoting cell growth and metabolism, they also cause various systemic side effects.

Since RARγ is mainly expressed in the skin, highly selective RARγ agonists have become a research hotspot in recent years. Thoreau et al. reported in patent US8362082B2 RARγ selective agonists such as compound 10Y having the following structure.

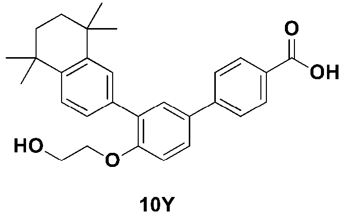

Although these compounds can appropriately improve selectivity relative to RARα and RARβ, according to literature reports and studies, they have certain agonist activity against RARα and/or RARβ, leading to systemic or other tissue side effects. In addition, existing RARγ selective agonist compounds of this type have a full agonist effect on RARγ similar to retinoic acid, posing a risk of adverse reactions caused by full activation, causing significant skin irritation, and topical application results in severe adverse reactions such as erythema, desquamation, and stinging. Therefore, there is still a need to develop novel RARγ selective agonists with better selectivity and/or reduced risk of overactivation and/or low irritation.

### SUMMARY

The present disclosure provides a biphenyl compound, a preparation method therefor, an intermediate thereof, a pharmaceutical composition thereof, and a use thereof. Such compounds have good retinoic acid receptor γ agonist activity and retinoic acid receptor γ selectivity, with little to no agonist activity against retinoic acid receptor α and/or retinoic acid receptor β, which is beneficial for reducing systemic side effects; more preferably, they have moderate partial agonist activity, resulting in low skin irritation, few adverse reactions, and excellent safety and compliance while maintaining efficacy.

In one aspect, the present disclosure provides a compound of formula I or a pharmaceutically acceptable salt thereof, 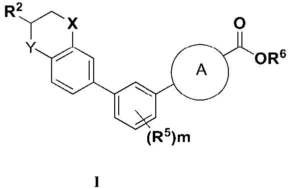
A is 6- to 10-membered arylene or 5- to 6-membered heteroarylene; the 5- to 6-membered heteroarylene has 1, 2, or 3 heteroatoms independently selected from the group consisting of N, O, and S;
Y is -NR¹- or -CR¹R'-;
R¹ is H or C₁₋₆ alkyl;
R¹ is H or C₁₋₆ alkyl;
R² is H or C₁₋₆ alkyl; or, R¹ and R², together with the intervening atom, form a 3- to 8-membered heterocycle or a C₃₋₈ carbocycle; the 3- to 8-membered heterocycle has 1, 2, or 3 heteroatoms independently selected from the group consisting of N, O, and S; the 3- to 8-membered heterocycle and C₃₋₈ carbocycle are optionally substituted with one or more R²⁻¹; each R²⁻¹ is independently C₁₋₆ alkyl, C₁₋₆ alkoxy, or halogen;
X is -NR³- or -CR³R⁴-; R³ is H or C₁₋₆ alkyl; R⁴ is H or C₁₋₆ alkyl;
R⁵ is H, C₁₋₆ alkyl, C₁₋₆ alkyl substituted with one or more R^{5a}, C₁₋₆ alkoxy, or C₁₋₆ alkoxy substituted with one or more R^{5a};
each R^{5a} is independently halogen or hydroxyl;
m is 0, 1, 2, or 3;
R⁶ is H or C₁₋₆ alkyl;
when Y is -CR¹R'-, R¹ and R², together with the intervening atom, form a 3- to 8-membered heterocycle or a C₃₋₈ carbocycle; the 3- to 8-membered heterocycle has 1, 2, or 3 heteroatoms independently selected from the group consisting of N, O, and S.

In some embodiments, in the compound of formula I or the pharmaceutically acceptable salt thereof, certain groups have the following definitions, and the definitions of groups not mentioned are as described in any embodiment of the present disclosure (hereinafter referred to as "in some embodiments").

In some embodiments, in A, the 6- to 10-membered arylene is phenylene or naphthylene, for example, phenylene.

In some embodiments, in A, the 5- to 6-membered heteroarylene may have 1 heteroatom, and the heteroatom is preferably N, for example, pyridylene, and for another example, 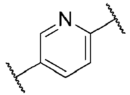 ( 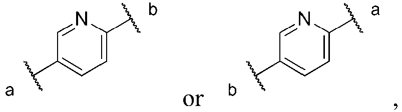 wherein the a end is connected to phenylene, and the b end is connected to carbonyl).

In some embodiments, in R¹, the C₁₋₆ alkyl may be methyl, ethyl, *n*-propyl, isopropyl, *n*-butyl, isobutyl, *sec*-butyl, or *tert*-butyl, for example, methyl or ethyl.

In some embodiments, in R', the C₁₋₆ alkyl may be methyl, ethyl, *n*-propyl, isopropyl, *n*-butyl, isobutyl, *sec*-butyl, or *tert*-butyl, for example, methyl or ethyl.

In some embodiments, in R², the C₁₋₆ alkyl may be methyl, ethyl, *n*-propyl, isopropyl, *n*-butyl, isobutyl, *sec*-butyl, or *tert*-butyl, for example, methyl or ethyl.

In some embodiments, in the C₃₋₈ carbocycle formed by R¹ and R² together with the intervening atom, the C₃₋₈ carbocycle may be monocyclic, such as C₄₋₆ monocyclic carbocycle, for example, cyclopentane (5-membered carbocycle).

In some embodiments, in the 3- to 8-membered heterocycle formed by R¹ and R² together with the intervening atom, the 3- to 8-membered heterocycle may have 1 heteroatom, and the heteroatom is preferably N; and/or may be monocyclic; the 3- to 8-membered heterocycle is preferably a 4- to 6-membered heterocycle, more preferably a 4- to 6-membered monocyclic heterocycle; for example,

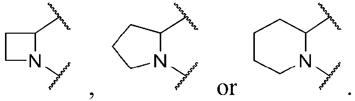

In some embodiments, in R³, the C₁₋₆ alkyl may be methyl, ethyl, *n*-propyl, isopropyl, *n*-butyl, isobutyl, *sec*-butyl, or *tert*-butyl, for example, methyl.

In some embodiments, in R⁴, the C₁₋₆ alkyl may be methyl, ethyl, *n*-propyl, isopropyl, *n*-butyl, isobutyl, *sec*-butyl, or *tert*-butyl, for example, methyl.

In some embodiments, in R⁵, the C₁₋₆ alkyl may be methyl, ethyl, *n*-propyl, isopropyl, *n*-butyl, isobutyl, *sec*-butyl, or *tert-butyl.*

In some embodiments, in R⁵, the C₁₋₆ alkoxy may be methoxy, ethoxy, *n*-propoxy, isopropoxy, *n*-butoxy, isobutoxy, *sec*-butoxy, or *tert*-butoxy, for example, ethoxy, *n*-propoxy, or *n*-butoxy.

In some embodiments, in R^{5a}, the halogen may be fluorine, chlorine, bromine, or iodine.

In some embodiments, in R⁵, the C₁₋₆ alkyl substituted with one or more R^{5a} is C₁₋₆ alkyl substituted with one R^{5a}, for example, C₁₋₆ alkyl substituted with one hydroxyl.

In some embodiments, in R⁵, the C₁₋₆ alkoxy substituted with one or more R^{5a} may be C₁₋₆ alkoxy substituted with one R^{5a}, for example, C₁₋₆ alkoxy substituted with one hydroxyl, and for another example,

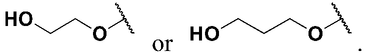

In some embodiments, in R⁶, the C₁₋₆ alkyl may be methyl, ethyl, *n*-propyl, isopropyl, *n*-butyl, isobutyl, *sec*-butyl, or *tert*-butyl.

In some embodiments, in R²⁻¹, the C₁₋₆ alkyl may be methyl, ethyl, *n*-propyl, isopropyl, *n*-butyl, isobutyl, *sec*-butyl, or *tert*-butyl.

In some embodiments, in R²⁻¹, the C₁₋₆ alkoxy may be methoxy, ethoxy, *n*-propoxy, isopropoxy, *n*-butoxy, isobutoxy, *sec*-butoxy, or *tert*-butoxy.

In some embodiments, in R²⁻¹, the halogen may be fluorine, chlorine, bromine, or iodine.

In some embodiments, the more than one refers to 2 or 3.

In some embodiments, m is 0, 1, or 2, preferably, m is 1.

In some embodiments, A is 6- to 10-membered arylene or 5- to 6-membered heteroarylene; the 5- to 6-membered heteroarylene has 1 heteroatom, and the heteroatom is N; preferably, A is 6- to 10-membered arylene; more preferably, A is phenylene.

In some embodiments, Y is -NR¹- or -CHR¹-; preferably, Y is -NR¹-.

In some embodiments, R¹ is H or C₁₋₆ alkyl; R² is H or C₁₋₆ alkyl; or, R¹ and R², together with the intervening atom, form a 3- to 8-membered heterocycle or a C₃₋₈ carbocycle; the 3- to 8-membered heterocycle has 1, 2, or 3 heteroatoms independently selected from the group consisting of N, O, and S; preferably, R¹ is H or C₁₋₆ alkyl; R² is H or C₁₋₆ alkyl; or, R¹ and R², together with the intervening atom, form a 3- to 8-membered heterocycle (for example, a 4- to 6-membered heterocycle) or a C₃₋₈ carbocycle (for example, a C₄₋₆ carbocycle); the 3-to 8-membered heterocycle is monocyclic and has 1 heteroatom, and the heteroatom is N; the C₃₋₈ carbocycle is monocyclic; more preferably, R¹ is C₁₋₆ alkyl; R² is H or C₁₋₆ alkyl; or, R¹ and R², together with the intervening atom, form a 3- to 8-membered heterocycle (for example, a 4- to 6-membered heterocycle) or a C₃₋₈ carbocycle (for example, a C₄₋₆ carbocycle); the 3-to 8-membered heterocycle is monocyclic and has 1 heteroatom, and the heteroatom is N; the C₃₋₈ carbocycle is monocyclic.

In some embodiments, X is -CR³R⁴-.

In some embodiments, R³ is C₁₋₆ alkyl.

In some embodiments, R⁴ is C₁₋₆ alkyl.

In some embodiments, R⁵ is C₁₋₆ alkoxy or C₁₋₆ alkoxy substituted with one or more R^{5a}, preferably, R⁵ is C₁₋₆ alkoxy substituted with one R^{5a}.

In some embodiments, R^{5a} is fluorine, chlorine, bromine, or hydroxyl, preferably, R^{5a} is hydroxyl.

In some embodiments, R⁶ is H, methyl, ethyl, *n*-propyl, isopropyl, *n*-butyl, isobutyl, *sec*-butyl, or *tert-butyl,* preferably, R⁶ is H.

In some embodiments, when Y is -CHR¹-, R¹ and R², together with the intervening atom, form a 3- to 8-membered heterocycle or a C₃₋₈ carbocycle; the 3- to 8-membered heterocycle has 1, 2, or 3 heteroatoms independently selected from the group consisting of N, O, and S; preferably, when Y is -CHR¹-, R¹ and R², together with the intervening atom, form a 3- to 8-membered heterocycle or a C₃₋₈ carbocycle; the 3- to 8-membered heterocycle is monocyclic and has 1 heteroatom, and the heteroatom is N; the C₃₋₈ carbocycle is monocyclic (for example, the C₄₋₆ monocyclic carbocycle); more preferably, when Y is -CHR¹-, R¹ and R², together with the intervening atom, form a C₃₋₈ carbocycle; the C₃₋₈ carbocycle is monocyclic.

In some embodiments, when Y is -NR¹-, R¹ and R², together with the intervening atom, form a 3- to 8-membered heterocycle; the 3- to 8-membered heterocycle is optionally substituted with one or more R²⁻¹; each R²⁻¹ is independently C₁₋₆ alkyl, C₁₋₆ alkoxy, or halogen; the 3- to 8-membered heterocycle has 1, 2, or 3 heteroatoms independently selected from the group consisting of N, O, and S, wherein at least one of the heteroatoms is N; preferably, when Y is -NR¹-, R¹ and R², together with the intervening atom, form a 3- to 8-membered heterocycle; the 3- to 8-membered heterocycle is optionally substituted with one or more R²⁻¹; each R²⁻¹ is independently C₁₋₆ alkyl, C₁₋₆ alkoxy, or halogen; the 3- to 8-membered heterocycle is monocyclic and has 1 heteroatom, and the heteroatom is N.

In some embodiments,
m is 0, 1, or 2;
A is 6- to 10-membered arylene or 5- to 6-membered heteroarylene; the 5- to 6-membered heteroarylene has 1 heteroatom, and the heteroatom is N;
Y is -NR¹- or -CHR¹-;
R¹ is H or C₁₋₆ alkyl; R² is H or C₁₋₆ alkyl; or, R¹ and R², together with the intervening atom, form a 3- to 8-membered heterocycle (for example, a 4- to 6-membered heterocycle) or a C₃₋₈ carbocycle (for example, a C₄₋₆ carbocycle); the 3- to 8-membered heterocycle (for example, the 4- to 6-membered heterocycle) is monocyclic and has 1 heteroatom, and the heteroatom is N; the C₃₋₈ carbocycle (for example, the C₄₋₆ carbocycle) is monocyclic;
X is -NR³- or -CR³R⁴-;
R³ is H or C₁₋₆ alkyl;
R⁴ is H or C₁₋₆ alkyl;
R⁵ is C₁₋₆ alkoxy or C₁₋₆ alkoxy substituted with one or more R^{5a};
R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl;
when Y is -CHR¹-, R¹ and R², together with the intervening atom, form a C₃₋₈ carbocycle; the C₃₋₈ carbocycle is monocyclic (for example, C₄₋₆ monocyclic carbocycle).

In some embodiments,
m is 0, 1, or 2;
A is 6- to 10-membered arylene;
Y is -NR¹-;
R¹ is H or C₁₋₆ alkyl; R² is H or C₁₋₆ alkyl; or, R¹ and R², together with the intervening atom, form a 3- to 8-membered heterocycle (for example, a 4- to 6-membered heterocycle); the 3- to 8-membered heterocycle (for example, the 4- to 6-membered heterocycle) is monocyclic and has 1 heteroatom, and the heteroatom is N;
X is -CR³R⁴-;
R³ is H or C₁₋₆ alkyl;
R⁴ is H or C₁₋₆ alkyl;
R⁵ is C₁₋₆ alkoxy or C₁₋₆ alkoxy substituted with one or more R^{5a};
R^{5a} is fluorine, chlorine, bromine, or hydroxyl;
R⁶ is H, methyl, ethyl, n-propyl, isopropyl, n-butyl, isobutyl, sec-butyl, or tert-butyl.

In some embodiments,
m is 1;
A is 6- to 10-membered arylene or 5- to 6-membered heteroarylene; the 5- to 6-membered heteroarylene has 1 heteroatom, and the heteroatom is N;
Y is -NR¹- or -CHR¹-;
R¹ is H or C₁₋₆ alkyl; R² is H or C₁₋₆ alkyl; or, R¹ and R², together with the intervening atom, form a 3- to 8-membered heterocycle (for example, a 4- to 6-membered heterocycle) or a C₃₋₈ carbocycle (for example, a C₄₋₆ carbocycle); the 3- to 8-membered heterocycle (for example, the 4- to 6-membered heterocycle) is monocyclic and has 1 heteroatom, and the heteroatom is N; the C₃₋₈ carbocycle (for example, the C₄₋₆ carbocycle) is monocyclic;
X is -NR³- or -CR³R⁴-; R³ is C₁₋₆ alkyl; R⁴ is C₁₋₆ alkyl;
R⁵ is C₁₋₆ alkoxy substituted with one R^{5a};
R^{5a} is hydroxyl;
R⁶ is H;
when Y is -CHR¹-, R¹ and R², together with the intervening atom, form a C₃₋₈ carbocycle (for example, a C₄₋₆ carbocycle), and the C₃₋₈ carbocycle (for example, the C₄₋₆ carbocycle) is monocyclic.

In some embodiments,
m is 1;
A is 6- to 10-membered arylene;
Y is -NR¹-;
R¹ is C₁₋₆ alkyl; R² is H or C₁₋₆ alkyl; or, R¹ and R², together with the intervening atom, form a 3- to 8-membered heterocycle (for example, a 4- to 6-membered heterocycle); the 3- to 8-membered heterocycle (for example, the 4- to 6-membered heterocycle) is monocyclic and has 1 heteroatom, and the heteroatom is N;
X is -CR³R⁴-; R³ is C₁₋₆ alkyl; R⁴ is C₁₋₆ alkyl;
R⁵ is C₁₋₆ alkoxy substituted with one R^{5a};
R^{5a} is hydroxyl;
R⁶ is H.

In some embodiments, the compound of formula I may be a compound of formula I-1 or a compound of formula I-2: 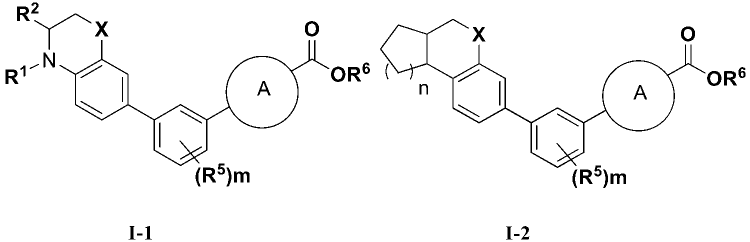 (for example, 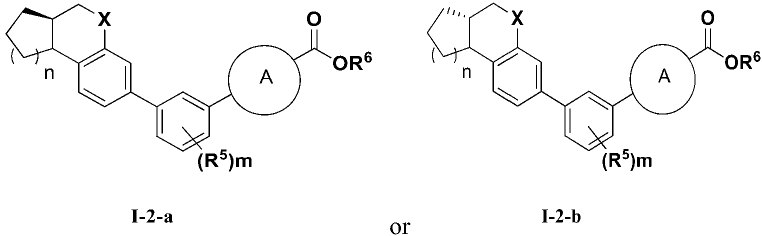 );
n is 0, 1, 2, or 3; m, A, R¹, R², X, R⁵, and R⁶ are as defined above.

In some embodiments, in the compound of formula I-1,
m is 0, 1, or 2;
A is 6- to 10-membered arylene;
R¹ is H or C₁₋₆ alkyl; R² is H or C₁₋₆ alkyl; or, R¹ and R², together with the intervening atom, form a 3- to 8-membered heterocycle (for example, a 4- to 6-membered heterocycle); the 3- to 8-membered heterocycle (for example, the 4- to 6-membered heterocycle) is monocyclic and has 1 heteroatom, and the heteroatom is N;
X is -CR³R⁴-; R³ is H or C₁₋₆ alkyl; R⁴ is H or C₁₋₆ alkyl;
R⁵ is C₁₋₆ alkoxy or C₁₋₆ alkoxy substituted with one or more R^{5a};
R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl.

In some embodiments, in the compound of formula I-1,
m is 1; A is phenylene;
R¹ is C₁₋₆ alkyl; R² is C₁₋₆ alkyl or H; or, R¹ and R², together with the intervening atom, form a 3- to 8-membered heterocycle (for example, a 4- to 6-membered heterocycle); the 3- to 8-membered heterocycle (for example, the 4- to 6-membered heterocycle) is monocyclic and has 1 heteroatom, and the heteroatom is N;
X is -CR³R⁴-; R³ is C₁₋₆ alkyl; R⁴ is C₁₋₆ alkyl;
R⁵ is C₁₋₆ alkoxy substituted with one R^{5a};
R^{5a} is hydroxyl;
R⁶ is H.

In some embodiments, in the compound of formula I-2 (for example, compound of I-2-a or I-2-b),
n is 0, 1, or 2;
m is 0, 1, or 2;
A is 6- to 10-membered arylene or 5- to 6-membered heteroarylene; the 5- to 6-membered heteroarylene has 1 heteroatom, and the heteroatom is N;
X is -NR³- or -CR³R⁴-; R³ is H or C₁₋₆ alkyl; R⁴ is H or C₁₋₆ alkyl;
R⁵ is C₁₋₆ alkoxy or C₁₋₆ alkoxy substituted with one or more R^{5a};
R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl.

In some embodiments, in the compound of formula I-2 (for example, compound of I-2-a or I-2-b),
n is 1; m is 1; A is phenylene;
X is -CR³R⁴-; R³ is C₁₋₆ alkyl; R⁴ is C₁₋₆ alkyl;
R⁵ is C₁₋₆ alkoxy substituted with one R^{5a};
R^{5a} is hydroxyl;
R⁶ is H.

In some embodiments, the compound of formula I may be a compound of formula I-1-a, a compound of formula I-1-b, or a compound of formula I-1-c: 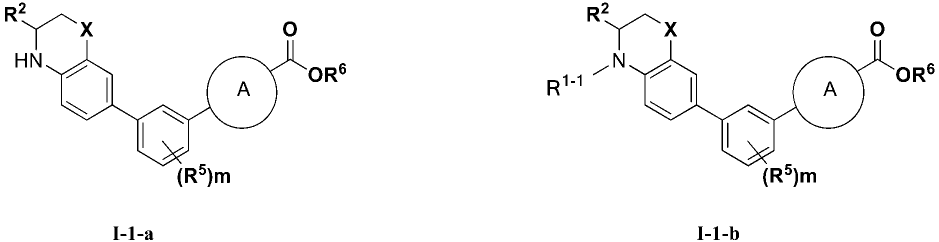 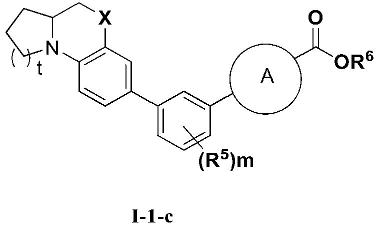 (for example, 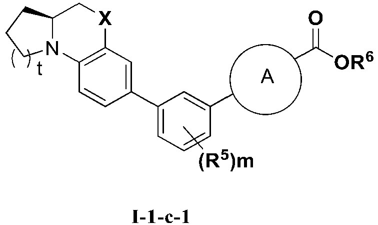 or 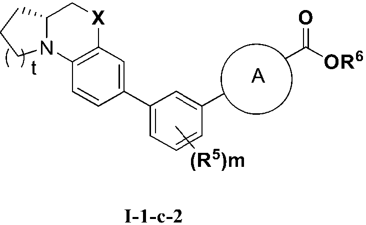 );
t is 0, 1, 2, or 3; R¹⁻¹ is C₁₋₆ alkyl; m, A, R², X, R⁵, and R⁶ are as defined above. In R¹⁻¹, the C₁₋₆ alkyl is preferably methyl, ethyl, *n*-propyl, isopropyl, *n*-butyl, isobutyl, *sec*-butyl, or *tert*-butyl, for example, methyl or ethyl.

In some embodiments, in the compound of formula I-1-a,
m is 0, 1, or 2;
A is 6- to 10-membered arylene;
R² is H or C₁₋₆ alkyl;
X is -CR³R⁴-; R³ is H or C₁₋₆ alkyl; R⁴ is H or C₁₋₆ alkyl;
R⁵ is C₁₋₆ alkoxy or C₁₋₆ alkoxy substituted with one or more R^{5a};
R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl.

In some embodiments, in the compound of formula I-1-a,
m is 1; A is phenylene;
R² is H or C₁₋₆ alkyl; X is -CR³R⁴-; R³ is C₁₋₆ alkyl; R⁴ is C₁₋₆ alkyl;
R⁵ is C₁₋₆ alkoxy substituted with one R^{5a};
R^{5a} is hydroxyl;
R⁶ is H.

In some embodiments, in the compound of formula I-1-b,
m is 0, 1, or 2;
A is 6- to 10-membered arylene;
R¹⁻¹ is C₁₋₆ alkyl;
R² is H or C₁₋₆ alkyl;
X is -CR³R⁴-; R³ is H or C₁₋₆ alkyl; R⁴ is H or C₁₋₆ alkyl;
R⁵ is C₁₋₆ alkoxy or C₁₋₆ alkoxy substituted with one or more R^{5a};
R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl.

In some embodiments, in the compound of formula I-1-b,
m is 1; A is phenylene;
R¹⁻¹ is C₁₋₆ alkyl;
R² is H or C₁₋₆ alkyl;
X is -CR³R⁴-; R³ is C₁₋₆ alkyl; R⁴ is C₁₋₆ alkyl;
R⁵ is C₁₋₆ alkoxy substituted with one R^{5a};
R^{5a} is hydroxyl;
R⁶ is H.

In some embodiments, in the compound of formula I-1-c (for example, compound of I-1-c-1 or I-1-c-2),
t is 0, 1, or 2;
m is 0, 1, or 2;
A is 6- to 10-membered arylene;
X is -CR³R⁴-; R³ is H or C₁₋₆ alkyl; R⁴ is H or C₁₋₆ alkyl;
R⁵ is C₁₋₆ alkoxy or C₁₋₆ alkoxy substituted with one or more R^{5a};
R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl.

In some embodiments, in the compound of formula I-1-c (for example, compound of I-1-c-1 or I-1-c-2),
t is 1 or 2; m is 0 or 1; A is phenylene;
X is -CR³R⁴-; R³ is C₁₋₆ alkyl; R⁴ is C₁₋₆ alkyl;
R⁵ is C₁₋₆ alkoxy substituted with one or more R^{5a};
R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl.

In some embodiments, in the compound of formula I-1-c (for example, compound of I-1-c-1 or I-1-c-2),
t is 1; m is 1; A is phenylene;
X is -CR³R⁴-; R³ is C₁₋₆ alkyl; R⁴ is C₁₋₆ alkyl;
R⁵ is C₁₋₆ alkoxy substituted with one R^{5a};
R^{5a} is hydroxyl;
R⁶ is H.

In some embodiments, the compound of formula I may be a compound of formula I-1-1 or a compound of formula I-2-1: 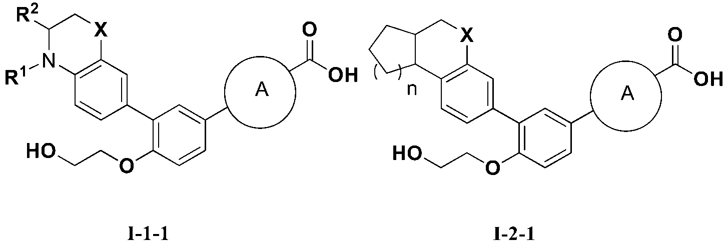 (for example, 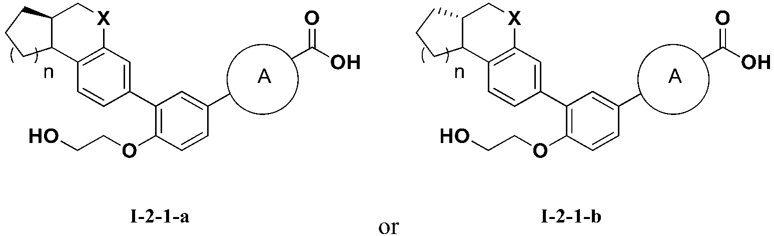 )
n is 0, 1, 2, or 3; A, R¹, R², and X are as defined above.

In some embodiments, in the compound of formula I-1-1,
A is 6- to 10-membered arylene;
R¹ is H or C₁₋₆ alkyl; R² is H or C₁₋₆ alkyl; or, R¹ and R², together with the intervening atom, form a 3- to 8-membered heterocycle (for example, a 4- to 6-membered heterocycle); the 3- to 8-membered heterocycle (for example, the 4- to 6-membered heterocycle) is monocyclic and has 1 heteroatom, and the heteroatom is N;
X is -CR³R⁴-; R³ is H or C₁₋₆ alkyl; R⁴ is H or C₁₋₆ alkyl.

In some embodiments, in the compound of formula I-1-1,
A is phenylene;
R¹ is C₁₋₆ alkyl; R² is H or C₁₋₆ alkyl; or, R¹ and R², together with the intervening atom, form a 3- to 8-membered heterocycle; the 3- to 8-membered heterocycle (for example, the 4-to 6-membered heterocycle) is monocyclic and has 1 heteroatom, and the heteroatom is N;
X is -CR³R⁴-; R³ is C₁₋₆ alkyl; R⁴ is C₁₋₆ alkyl.

In some embodiments, in the compound of formula I-2-1 (for example, I-2-1-a, I-2-1-b),
n is 0, 1, or 2; A is 6- to 10-membered arylene;
X is -CR³R⁴-; R³ is H or C₁₋₆ alkyl; R⁴ is H or C₁₋₆ alkyl.

In some embodiments, in the compound of formula I-2-1 (for example, I-2-1-a, I-2-1-b),
n is 1; A is phenylene;
X is -CR³R⁴-; R³ is C₁₋₆ alkyl; R⁴ is C₁₋₆ alkyl.

In some embodiments, the compound of formula I may be a compound of formula I-1-1-1, a compound of formula I-1-1-2, or a compound of formula I-1-1-3: 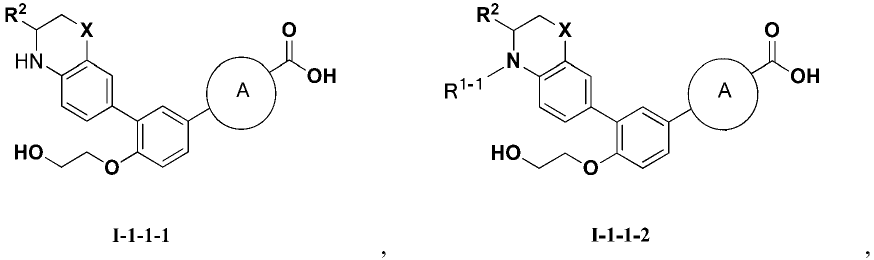 or 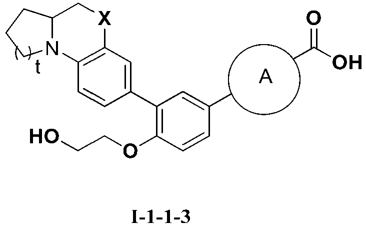 (for example, 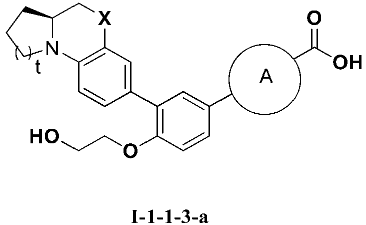 or 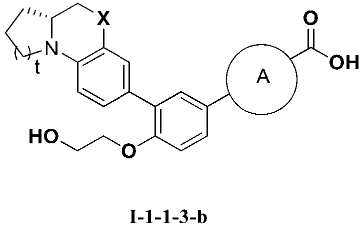 );
t is 0, 1, 2, or 3; R¹⁻¹ is C₁₋₆ alkyl; A, R², and X are as defined above. In R¹⁻¹, the C₁₋₆ alkyl is preferably methyl, ethyl, *n*-propyl, isopropyl, *n*-butyl, isobutyl, *sec*-butyl, or *tert*-butyl, for example, methyl or ethyl.

In some embodiments, in the compound of formula I-1-1-1,
A is 6- to 10-membered arylene; R² is H or C₁₋₆ alkyl; X is -CR³R⁴-; R³ is H or C₁₋₆ alkyl; R⁴ is H or C₁₋₆ alkyl.

In some embodiments, in the compound of formula I-1-1-1,
A is phenylene; R² is H or C₁₋₆ alkyl; X is -CR³R⁴-; R³ is C₁₋₆ alkyl; R⁴ is C₁₋₆ alkyl.

In some embodiments, in the compound of formula I-1-1-2,
A is 6- to 10-membered arylene; R¹⁻¹ is C₁₋₆ alkyl; R² is H or C₁₋₆ alkyl; X is -CR³R⁴-; R³ is H or C₁₋₆ alkyl; R⁴ is H or C₁₋₆ alkyl.

In some embodiments, in the compound of formula I-1-1-2,
A is phenylene; R¹⁻¹ is C₁₋₆ alkyl; R² is H or C₁₋₆ alkyl; X is -CR³R⁴-; R³ is C₁₋₆ alkyl; R⁴ is C₁₋₆ alkyl.

In some embodiments, in the compound of formula I-1-1-3 (for example, I-1-1-3-a, I-1-1-3-b),
t is 0, 1, or 2; A is 6- to 10-membered arylene; X is -CR³R⁴-; R³ is H or C₁₋₆ alkyl; R⁴ is H or C₁₋₆ alkyl.

In some embodiments, in the compound of formula I-1-1-3 (for example, I-1-1-3-a, I-1-1-3-b),
t is 1 or 2; A is phenylene; X is -CR³R⁴-; R³ is C₁₋₆ alkyl; R⁴ is C₁₋₆ alkyl.

In some embodiments, in the compound of formula I-1-1-3 (for example, I-1-1-3-a, I-1-1-3-b),
t is 1; A is phenylene; X is -CR³R⁴-; R³ is C₁₋₆ alkyl; R⁴ is C₁₋₆ alkyl.

In some embodiments, the compound of formula I may be a compound of formula I-A, a compound of formula I-B, a compound of formula I-C, or a compound of formula I-D 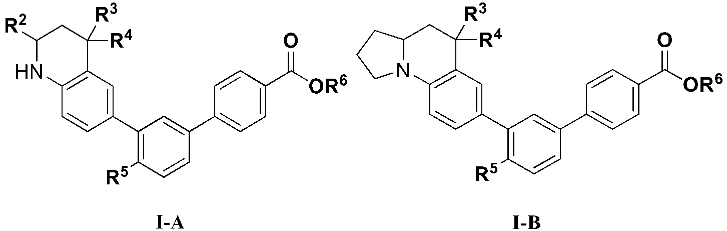 (for example, 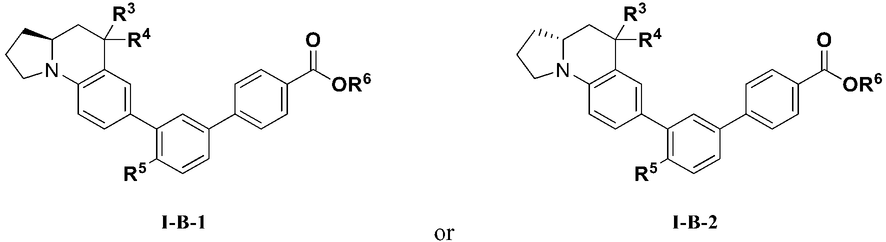 ) 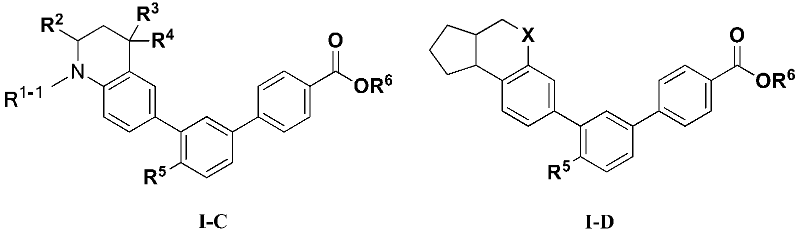 (for example, 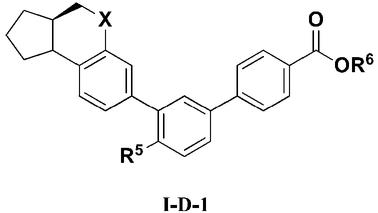 (corresponding to 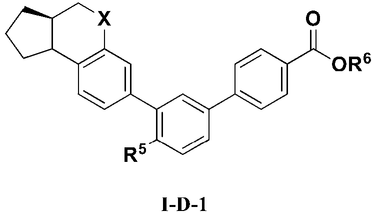 ) or 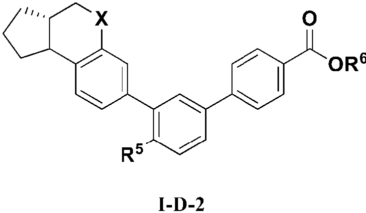 );
R¹⁻¹ is C₁₋₆ alkyl; X, R², R³, R⁴, R⁵, and R⁶ are as defined above. In R¹⁻¹, the C₁₋₆ alkyl is preferably methyl, ethyl, *n*-propyl, isopropyl, *n*-butyl, isobutyl, *sec*-butyl, or *tert*-butyl, for example, methyl or ethyl.

In some embodiments, in the compound of formula I-A,
R² is H or C₁₋₆ alkyl; R³ is H or C₁₋₆ alkyl; R⁴ is H or C₁₋₆ alkyl;
R⁵ is C₁₋₆ alkoxy or C₁₋₆ alkoxy substituted with one or more R^{5a}; R^{5a} is fluorine, chlorine, bromine, or hydroxyl;
R⁶ is H, methyl, ethyl, *n*-propyl, isopropyl, *n*-butyl, isobutyl, sec-butyl, or *tert*-butyl.

In some embodiments, in the compound of formula I-A,
R² is H or C₁₋₆ alkyl; R³ is C₁₋₆ alkyl; R⁴ is C₁₋₆ alkyl;
R⁵ is C₁₋₆ alkoxy substituted with one R^{5a}; R^{5a} is hydroxyl; R⁶ is H.

In some embodiments, in the compound of formula I-B (for example, I-B-1, I-B-2),
R³ is H or C₁₋₆ alkyl; R⁴ is H or C₁₋₆ alkyl;
R⁵ is C₁₋₆ alkoxy or C₁₋₆ alkoxy substituted with one or more R^{5a}; R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl.

In some embodiments, in the compound of formula I-B (for example, I-B-1, I-B-2),
R³ is C₁₋₆ alkyl; R⁴ is C₁₋₆ alkyl;
R⁵ is C₁₋₆ alkoxy substituted with one R^{5a}; R^{5a} is hydroxyl; R⁶ is H.

In some embodiments, in the compound of formula I-C,
R¹⁻¹ is C₁₋₆ alkyl;
R² is H or C₁₋₆ alkyl; R³ is H or C₁₋₆ alkyl; R⁴ is H or C₁₋₆ alkyl;
R⁵ is C₁₋₆ alkoxy or C₁₋₆ alkoxy substituted with one or more R^{5a}; R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl.

In some embodiments, in the compound of formula I-C,
R¹⁻¹ is C₁₋₆ alkyl; R² is H or C₁₋₆ alkyl; R³ is C₁₋₆ alkyl; R⁴ is C₁₋₆ alkyl;
R⁵ is C₁₋₆ alkoxy substituted with one R^{5a}; R^{5a} is hydroxyl; R⁶ is H.

In some embodiments, in the compound of formula I-D (for example, I-D-1, I-D-2),
X is -NR³- or -CR³R⁴-; R³ is H or C₁₋₆ alkyl; R⁴ is H or C₁₋₆ alkyl;
R⁵ is C₁₋₆ alkoxy or C₁₋₆ alkoxy substituted with one or more R^{5a}; R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl.

In some embodiments, in the compound of formula I-D (for example, I-D-1, I-D-2),
X is -CR³R⁴-; R³ is C₁₋₆ alkyl; R⁴ is C₁₋₆ alkyl; R⁵ is C₁₋₆ alkoxy substituted with one R^{5a}; R^{5a} is hydroxyl;
R⁶ is H.

In some embodiments, 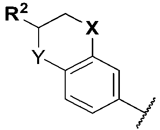 is 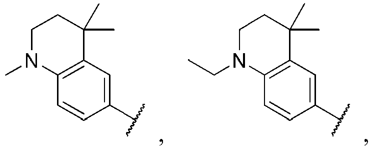 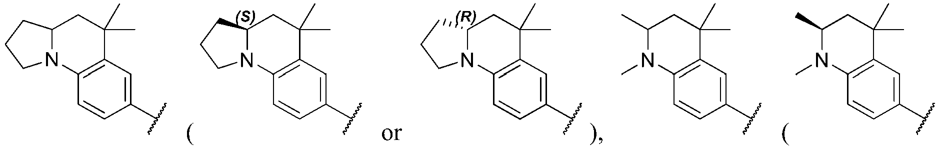 or

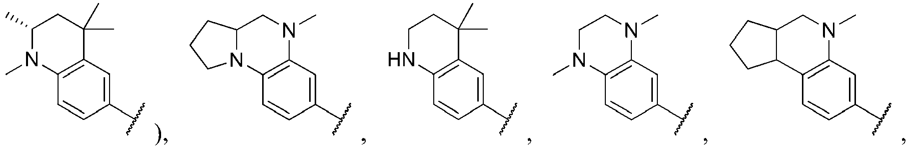

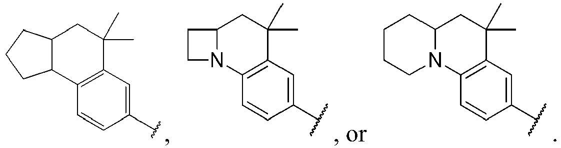

In some embodiments, R⁶ is hydrogen.

In some embodiments, R⁵ is

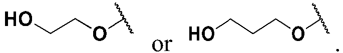

In some embodiments, 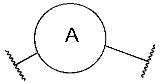 is 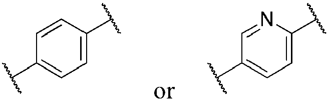 ( 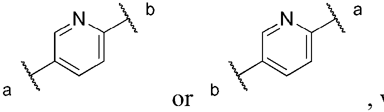 wherein the a end is connected to phenylene, and the b end is connected to carbonyl).

In some embodiments, the compound of formula I is any one of the following compounds:

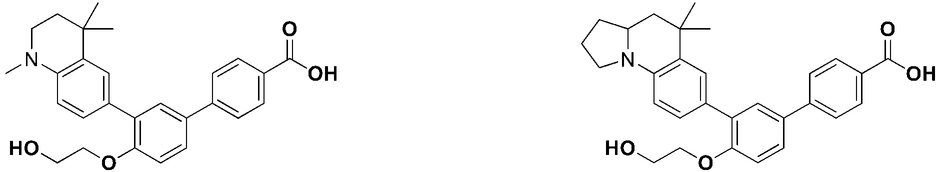

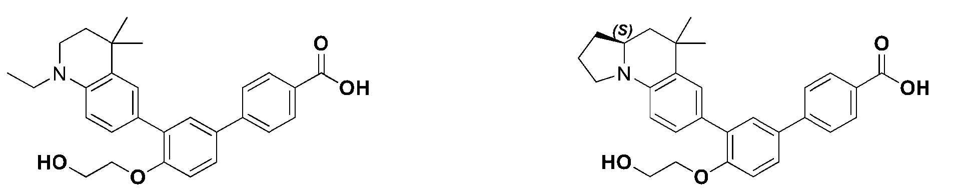

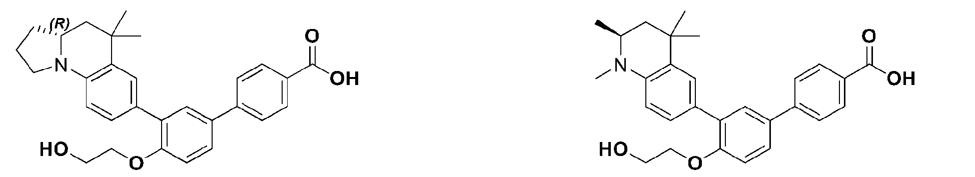

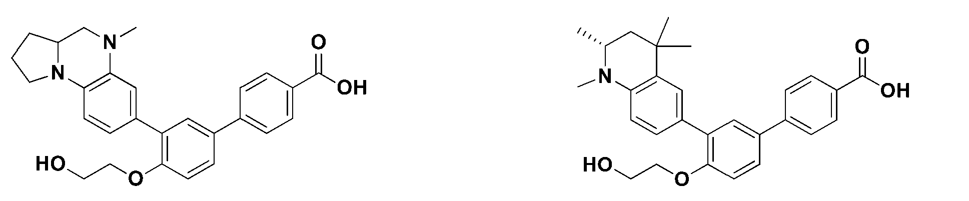

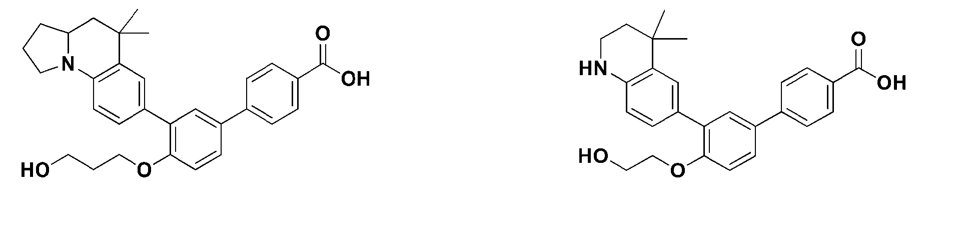

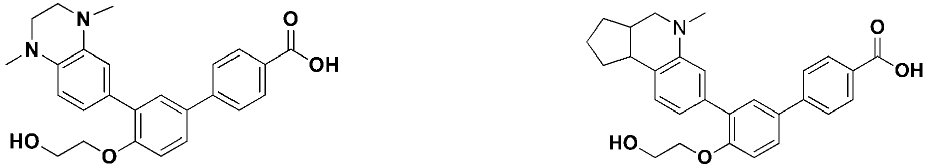

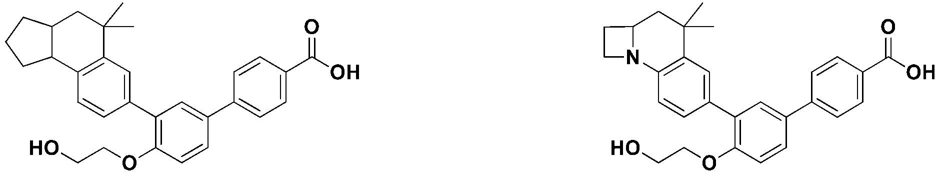

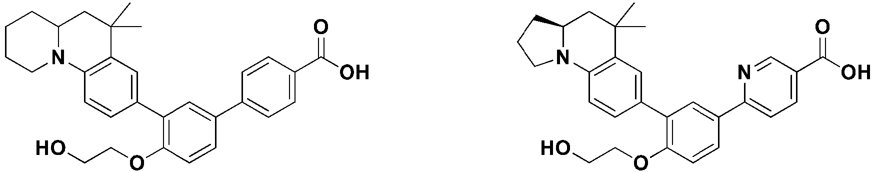

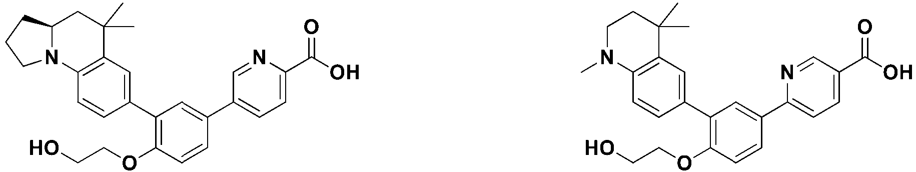

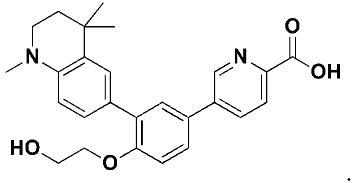

In another aspect, the present disclosure also provides a pharmaceutical composition comprising (i) the above compound of formula I or the pharmaceutically acceptable salt thereof; and (ii) a pharmaceutically acceptable carrier.

In another aspect, the present disclosure also provides a use of the above compound of formula I or the pharmaceutically acceptable salt thereof, or the above pharmaceutical composition, as a medicament.

In another aspect, the present disclosure also provides the above compound of formula I or the pharmaceutically acceptable salt thereof, or the above pharmaceutical composition, for use as a medicament.

In another aspect, the present disclosure also provides a use of the above compound of formula I or the pharmaceutically acceptable salt thereof, or the above pharmaceutical composition, in the manufacture of a medicament.

In another aspect, the present disclosure also provides a use of the above compound of formula I or the pharmaceutically acceptable salt thereof, or the above pharmaceutical composition, as a retinoic acid receptor (for example, retinoic acid receptor γ) agonist.

In another aspect, the present disclosure also provides a use of the above compound of formula I or the pharmaceutically acceptable salt thereof, or the above pharmaceutical composition, as a retinoic acid receptor (for example, retinoic acid receptor γ) partial agonist.

In another aspect, the present disclosure also provides the above compound of formula I or the pharmaceutically acceptable salt thereof, or the above pharmaceutical composition, for use as a retinoic acid receptor (for example, retinoic acid receptor γ) agonist.

In another aspect, the present disclosure also provides the above compound of formula I or the pharmaceutically acceptable salt thereof, or the above pharmaceutical composition, for use as a retinoic acid receptor (for example, retinoic acid receptor γ) partial agonist.

In another aspect, the present disclosure also provides a use of the above compound of formula I or the pharmaceutically acceptable salt thereof, or the above pharmaceutical composition, in the manufacture of a retinoic acid receptor (for example, retinoic acid receptor γ) agonist.

In another aspect, the present disclosure also provides a use of the above compound of formula I or the pharmaceutically acceptable salt thereof, or the above pharmaceutical composition, in the manufacture of a retinoic acid receptor (for example, retinoic acid receptor γ) partial agonist.

In another aspect, the present disclosure also provides the above compound of formula I or the pharmaceutically acceptable salt thereof, or the above pharmaceutical composition, for use in treating and/or preventing a disease.

In some embodiments, the disease is acne (including acne vulgaris, rosacea, nodulocystic acne, acne conglobata, and secondary acne caused by sunlight or medication), comedo, psoriasis, ichthyosis, cutaneous keratinization disorder, pigmentation, or dry eye syndrome.

In another aspect, the present disclosure also provides the above compound of formula I or the pharmaceutically acceptable salt thereof, or the above pharmaceutical composition, for use in treating and/or preventing a disease.

In some embodiments, the disease is acne (including acne vulgaris, rosacea, nodulocystic acne, acne conglobata, and secondary acne caused by sunlight or medication), comedo, psoriasis, ichthyosis, cutaneous keratinization disorder, pigmentation, or dry eye syndrome.

In another aspect, the present disclosure also provides a use of the above compound of formula I or the pharmaceutically acceptable salt thereof, or the above pharmaceutical composition, in the manufacture of a medicament for treating and/or preventing a disease.

In some embodiments, the disease is acne (including acne vulgaris, rosacea, nodulocystic acne, acne conglobata, and secondary acne caused by sunlight or medication), comedo, psoriasis, ichthyosis, cutaneous keratinization disorder, pigmentation, or dry eye syndrome.

In another aspect, the present disclosure also provides a use of the above compound of formula I or the pharmaceutically acceptable salt thereof, or the above pharmaceutical composition, as a medicament for treating and/or preventing a disease associated with a retinoic acid receptor (for example, retinoic acid receptor γ).

In some embodiments, the disease is acne (including acne vulgaris, rosacea, nodulocystic acne, acne conglobata, and secondary acne caused by sunlight or medication), comedo, psoriasis, ichthyosis, cutaneous keratinization disorder, pigmentation, or dry eye syndrome.

In another aspect, the present disclosure also provides the above compound of formula I or the pharmaceutically acceptable salt thereof, or the above pharmaceutical composition, for use in treating and/or preventing a disease associated with a retinoic acid receptor (for example, retinoic acid receptor γ).

In some embodiments, the disease is acne (including acne vulgaris, rosacea, nodulocystic acne, acne conglobata, and secondary acne caused by sunlight or medication), comedo, psoriasis, ichthyosis, cutaneous keratinization disorder, pigmentation, or dry eye syndrome.

In another aspect, the present disclosure also provides a use of the above compound of formula I or the pharmaceutically acceptable salt thereof, or the above pharmaceutical composition, in the manufacture of a medicament for treating and/or preventing a disease associated with a retinoic acid receptor (for example, retinoic acid receptor γ).

In some embodiments, the disease is acne (including acne vulgaris, rosacea, nodulocystic acne, acne conglobata, and secondary acne caused by sunlight or medication), comedo, psoriasis, ichthyosis, cutaneous keratinization disorder, pigmentation, or dry eye syndrome.

In another aspect, the present disclosure also provides a method of treating and/or preventing a disease associated with a retinoic acid receptor (for example, retinoic acid receptor γ), comprising administering to a subject in need thereof the above compound of formula I or the pharmaceutically acceptable salt thereof, or the above pharmaceutical composition.

In some embodiments, the disease is acne (including acne vulgaris, rosacea, nodulocystic acne, acne conglobata, and secondary acne caused by sunlight or medication), comedo, psoriasis, ichthyosis, cutaneous keratinization disorder, pigmentation, or dry eye syndrome.

In another aspect, the present disclosure also provides a compound of formula II or a salt thereof

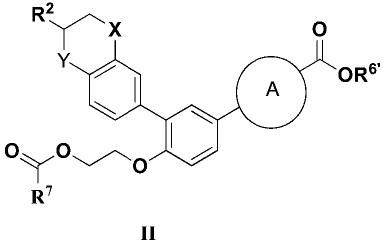

R^{6'} is C₁₋₆ alkyl; R⁷ is C₁₋₆ alkyl; A, R², X, and Y are as defined above.

In some embodiments, in R⁷, the C₁₋₆ alkyl may be methyl, ethyl, *n*-propyl, isopropyl, *n*-butyl, isobutyl, *sec*-butyl, or *tert*-butyl, for example, methyl.

In some embodiments, in R^{6'}, the C₁₋₆ alkyl may be methyl, ethyl, *n*-propyl, isopropyl, *n*-butyl, isobutyl, *sec*-butyl, or *tert*-butyl, for example, ethyl.

In some embodiments, the compound of formula II is any one of the following compounds:

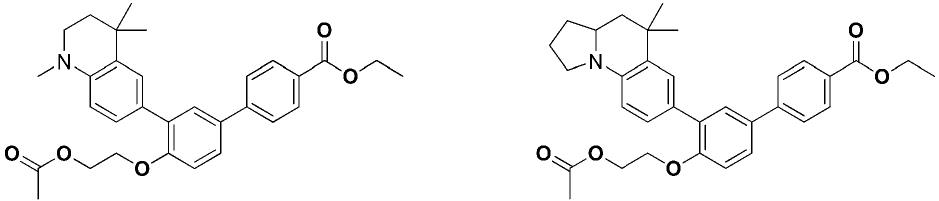

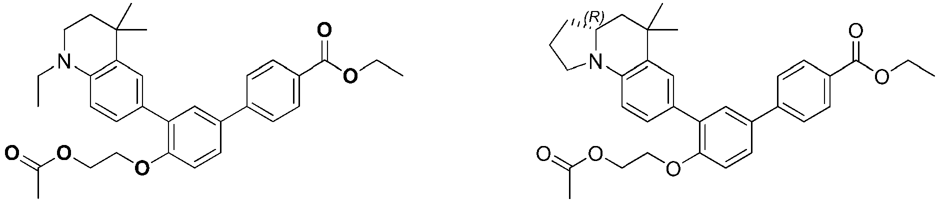

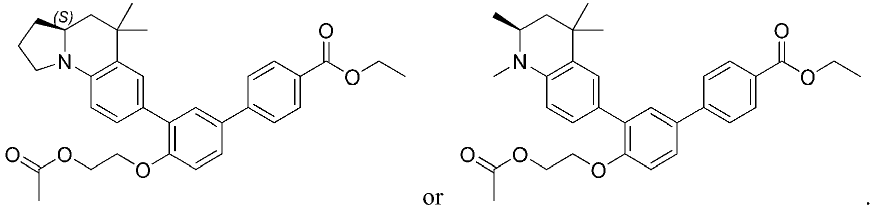

In another aspect, the present disclosure also provides a method for preparing a compound of formula I, comprising the following steps:

subjecting a compound of formula II to a hydrolysis reaction as shown below (for example, at 50-70°C, further for example, at 60°C) in an organic solvent (for example, an alcohol solvent, further for example, methanol) in the presence of a base (for example, an alkali metal hydroxide, further for example, sodium hydroxide, still further for example, 1 mol/L aqueous sodium hydroxide solution) to obtain the compound of formula I; 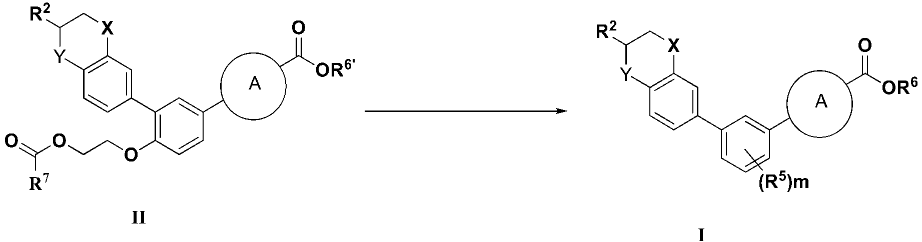 R⁶ is H; R⁵ is 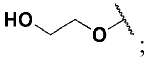 R⁵ is attached at the para position of A; m is 1; A, X, Y, R², R^{6'}, and R⁷ are as defined above.

In some embodiments, the method for preparing the compound of formula I further comprises the following step: subjecting a compound of formula III and a compound of formula IV to a coupling reaction as shown in the following scheme (for example, at 100-120°C, further for example, at 110°C) in a solvent (for example, a mixed solvent of water and a cyclic ether solvent, further for example, a mixed solvent of water and dioxane, still further for example, dioxane and water in a volume ratio of 20:3 or 4:1) in the presence of a catalyst (for example, a palladium catalyst, further for example, Pd(dppf)Cl₂) and an alkali metal halide salt (for example, an alkali metal fluoride salt, further for example, KF) to obtain the compound of formula II; 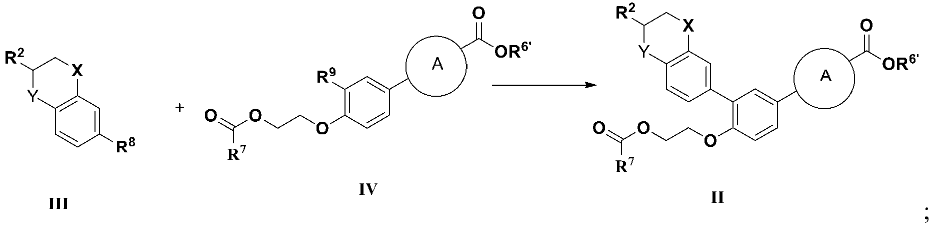 R⁸ is a boronate ester group 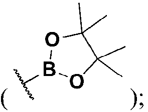 R⁹ is halogen (for example, bromine); A, X, Y, R², R^{6'}, and R⁷ are as defined above.

In another aspect, the present disclosure also provides a method for preparing a compound of formula II, comprising the following step: subjecting a compound of formula III and a compound of formula IV to a coupling reaction as shown in the following scheme (for example, at 100-120°C, further for example, at 110°C) in a solvent (for example, a mixed solvent of water and a cyclic ether solvent, further for example, a mixed solvent of water and dioxane, still further for example, dioxane and water in a volume ratio of 20:3 or 4:1) in the presence of a catalyst (for example, a palladium catalyst, further for example, Pd(dppf)Cl₂) and an alkali metal halide salt (for example, an alkali metal fluoride salt, further for example, KF) to obtain the compound of formula II; 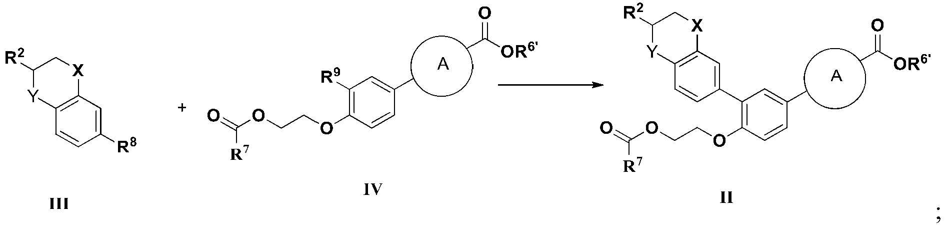 R⁸ is a boronate ester group 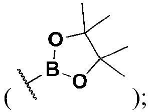 R⁹ is halogen (for example, bromine); A, X, Y, R², R^{6'}, and R⁷ are as defined above.

The positive and progressive effects of the present disclosure lie in that the biphenyl compound of the present disclosure (specifically, the compound of formula I or the pharmaceutically acceptable salt thereof) has good retinoic acid receptor γ agonist activity and high retinoic acid receptor γ selectivity, exhibits almost no agonist activity on retinoic acid receptor α and/or β, which is beneficial for reducing systemic side effects; more preferably, the biphenyl compound has moderate partial agonist activity. While maintaining efficacy, it exhibits low skin irritation, few adverse reactions, and excellent safety and compliance, and can be used for treating and preventing diseases associated with retinoic acid receptor γ.

### Definition of terms

Unless otherwise specified, the terms used in the present disclosure have the following definitions, and the definitions of terms not mentioned below are as commonly understood by those skilled in the art to which the present disclosure pertains.

In the present disclosure, the term "pharmaceutically acceptable salt" refers to a salt prepared from a compound with a relatively non-toxic, pharmaceutically acceptable acid or base. When the compound contains a relatively acidic functional group, a base addition salt can be obtained by contacting the neutral form of such compound with a sufficient amount of a pharmaceutically acceptable base in a pure solution or a suitable inert solvent. When the compound of the present disclosure contains a relatively basic functional group, an acid addition salt can be obtained by contacting the neutral form of such compound with a sufficient amount of a pharmaceutically acceptable acid in a pure solution or a suitable inert solvent. When the compound contains both relatively acidic and relatively basic functional groups, it can be converted into a base addition salt or an acid addition salt.

In the present disclosure, the term "more than one" refers to a natural number greater than 2, for example, 2, 3, 4, or 5.

In the present disclosure, the term "halogen" refers to fluorine, chlorine, bromine, or iodine.

In the present disclosure, the term "hydroxyl" refers to -OH.

In the present disclosure, the term "alkyl" refers to a saturated straight or branched monovalent hydrocarbon group having a certain number of carbon atoms. C₁₋₆ alkyl refers to an alkyl group having 1 to 6 (for example, 1, 2, 3, 4, 5, 6) carbon atoms, including C₁ alkyl, C₂ alkyl, C₃ alkyl, C₄ alkyl, C₅ alkyl, and C₆ alkyl, for example, methyl, ethyl, n-propyl, isopropyl, n-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like.

In the present disclosure, the term "alkoxy" refers to -O-alkyl, wherein alkyl is as defined above.

In the present disclosure, the term "carbocycle" refers to a saturated monocyclic or polycyclic ring having a specified number of carbon atoms. The term "C₃₋₈ carbocycle" refers to a saturated monocyclic or polycyclic ring having 3 to 8 (for example, 3, 4, 5, 6, 7, or 8) ring carbon atoms; preferably, C₃₋₈ carbocycle is a saturated monocyclic ring having 3 to 8 (for example, 3, 4, 5, 6, 7, or 8) ring carbon atoms, for example, C₃, C₄, C₅, or C₆ saturated monocyclic ring, specific examples include, but are not limited to, cyclopropane carbocycle (three-membered carbocycle), cyclobutane carbocycle (four-membered carbocycle), cyclopentane (five-membered carbocycle), cyclohexane carbocycle (six-membered carbocycle), and the like. Polycyclic rings may be spiro rings, fused rings, and bridged rings.

In the present disclosure, the term "heterocycle" refers to a saturated ring having a specified number of ring atoms (for example, 3- to 12-membered), a specified number of heteroatoms (for example, 1, 2, 3, or 4), and a specified type of heteroatoms (one or more of N, O, and S), including monocyclic or polycyclic rings. Polycyclic rings may be spiro rings, fused rings, and bridged rings.

In the present disclosure, the term "arylene" refers to a cyclic, unsaturated divalent hydrocarbon group having a specified number of carbon atoms (for example, C₆ to C₁₀), which is monocyclic or polycyclic (for example, 2 or 3 rings); when polycyclic, the individual rings share two atoms and one bond, and each ring is aromatic. Arylene rings include, but are not limited to, phenylene, naphthylene.

In the present disclosure, the term "heteroarylene" refers to a cyclic, unsaturated divalent group having a specified number of ring atoms (for example, 5- to 10-membered), a specified number of heteroatoms (for example, 1, 2, 3, or 4), and a specified type of heteroatoms (one or more of N, O, and S), which is monocyclic or polycyclic; when monocyclic, it may be pyridylene, further for example, 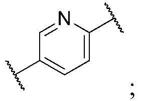 when polycyclic, the individual rings share two atoms and one bond, and each ring is aromatic; polycyclic refers to a fused ring formed by one heteroaromatic ring and one or more aromatic rings or heteroaromatic rings.

In the present disclosure, the term "substituted with..." means that one or more hydrogen atoms in a given structure are substituted with specific substituent(s). Further, when the group is substituted with more than one of the substituents, the substituents are independent of each other, i.e., the more than one substituent may be different from each other or may be the same. Unless otherwise explicitly indicated, a substituent group may be substituted at each substitutable position of the substituted group. When more than one position in a given structural formula can be substituted with one or more substituents selected from the group consisting of specific groups, the substituents may be substituted at each position identically or differently.

When a listed group does not explicitly indicate that it has a substituent, such group only mean unsubstituted. For example, when "C₁₋₆ alkyl" is not preceded by the limitation "unsubstituted or substituted", it refers only to "C₁₋₆ alkyl" itself or "unsubstituted C₁₋₆ alkyl".

In addition, it should be noted that, unless otherwise explicitly indicated, the descriptive manner "... be independently" used in the present disclosure should be understood in a broad sense, meaning that the described individuals are independent of each other and may independently be the same or different specific groups. More specifically, the descriptive manner "... be independently" may refer to that, in different groups, the specific options expressed by the same symbol do not affect each other; it may also mean that, in the same group, the specific options expressed by the same symbol do not affect each other.

In the present disclosure, the term "optionally substituted with ..." means either "substituted with ..." or "unsubstituted".

In the present disclosure, combinations of substituents and/or variants thereof are permissible only if such combinations result in reasonable compounds.

In the present disclosure, the term "treating" refers to therapeutic therapy. When referring to a specific condition, treating refers to: (1) alleviating one or more biological manifestations of the disease or condition, (2) interfering with (a) one or more points in the biological cascade that causes or leads to the condition or (b) one or more biological manifestations of the condition, (3) ameliorating one or more symptoms, effects, or side effects associated with the condition, or one or more symptoms, effects, or side effects associated with the condition or its treatment, or (4) slowing the progression of the condition or one or more biological manifestations of the condition.

In the present disclosure, the term "therapeutically effective amount" refers to an amount of a compound that, when administered to a patient, is sufficient to effectively treat or prevent the disease or condition described herein. The "therapeutically effective amount" will vary depending on the compound, the condition and its severity, and the age of the patient to be treated, but can be adjusted by those skilled in the art as needed. Dosages exceeding this range may also be used depending on the different dosage forms and the severity of the disease.

The above preferred conditions can be combined arbitrarily to obtain preferred embodiments of the present disclosure without violating common knowledge in the art.

The reagents and starting materials used in the present disclosure are all commercially available.

### BRIEF DESCRIPTION OF THE DRAWINGS

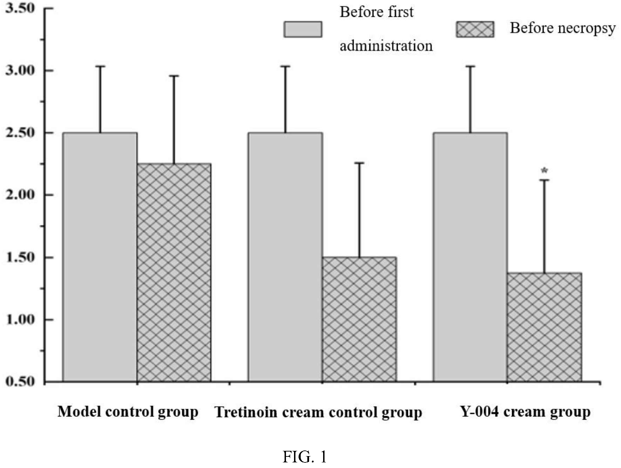

FIG. 1 shows the animal visual observation scores during the pharmacodynamic test of New Zealand rabbit ear acne model.

### DETAILED DESCRIPTION OF THE PREFERRED EMBODIMENT

The present disclosure will be described in detail below by way of examples, but the scope of the present disclosure is not limited thereto. Experimental methods that do not indicate specific conditions in the following examples should be selected according to conventional methods and conditions, or according to product specifications.

### General preparation method:

(1) subjecting a compound of formula III and a compound of formula IV or formula IV' to a coupling reaction as shown in the following scheme (for example, at 100-120°C, further for example, at 110°C) to obtain a compound of formula II or formula II' in a solvent (for example, a mixed solvent of water and a cyclic ether solvent, further for example, a mixed solvent of water and dioxane, still further for example, dioxane and water in a volume ratio of 20:3 or 4:1) in the presence of a catalyst (for example, a palladium catalyst, further for example, Pd(dppf)Cl₂) and an alkali metal halide salt (for example, an alkali metal fluoride salt, further for example, KF); 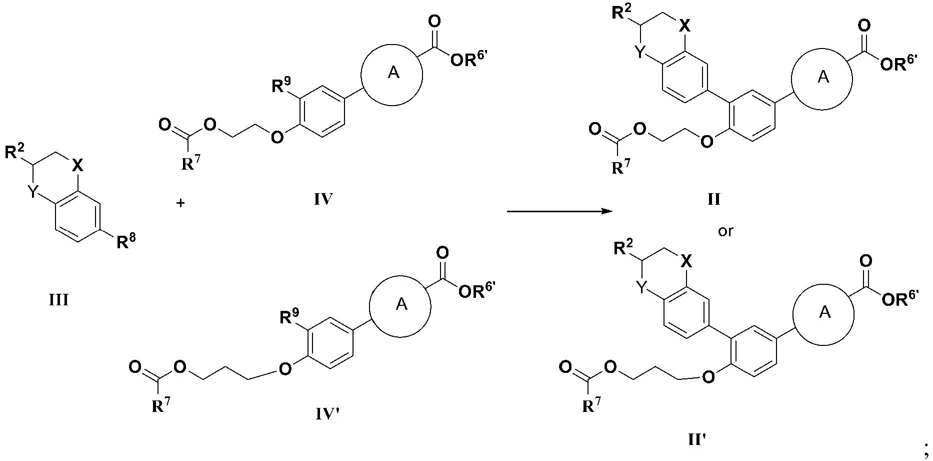 R⁸ is a boronate ester group 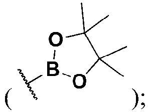 R⁹ is halogen (for example, bromine); A, X, Y, R², R^{6'}, and R⁷ are as defined above;
(2) subjecting a compound of formula II or a compound of formula II' to a hydrolysis reaction as shown below (for example, at 50-70°C, further for example, at 60°C) in an organic solvent (for example, an alcohol solvent, further for example, methanol) in the presence of a base (for example, an alkali metal hydroxide, further for example, sodium hydroxide, still further for example, 1 mol/L aqueous sodium hydroxide solution) to obtain the compound of formula I; 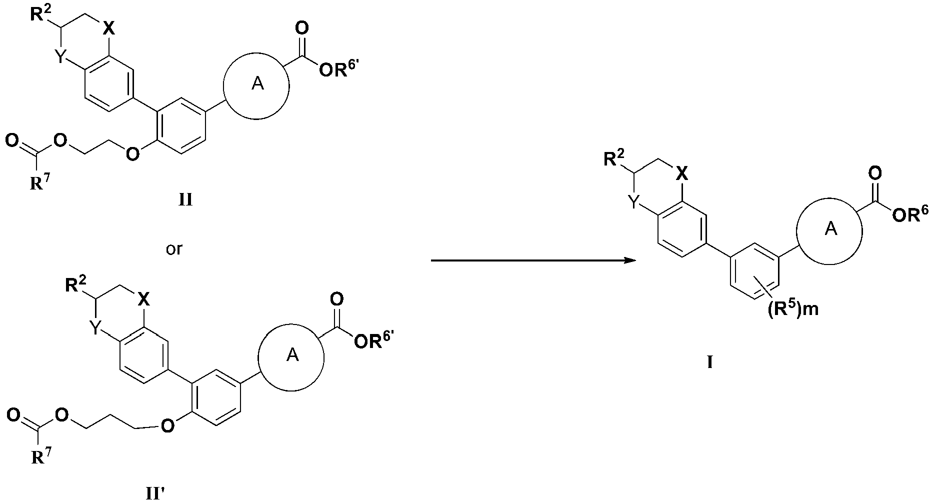 R⁶ is H; when the reactant is II, R⁵ is 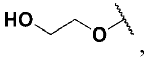 m is 1; when the reactant is II', R⁵ is 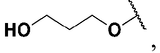 m is 1; R⁵ is attached at the para position of A; A, X, Y, R², R^{6'}, and R⁷ are as defined above.

### Example 1 Synthesis of compound Y-001:

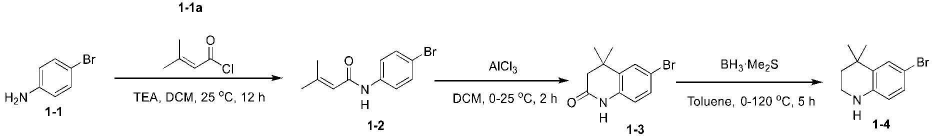

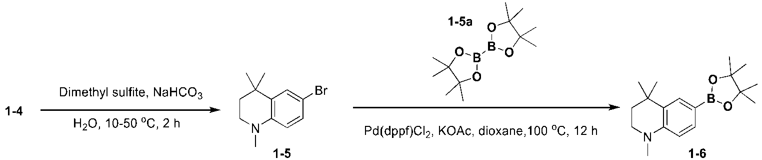

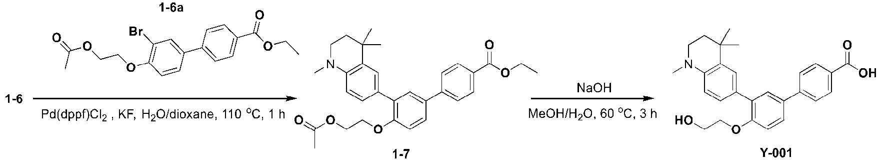

### Step 1: Synthesis of intermediate 1-2

Compound **1-1** (22 g, 127.89 mmol, 1 eq) was dissolved in 300 mL of dichloromethane, and triethylamine (31.99 g, 316.12 mmol, 2.47 eq) and **1-1a** (21.23 g, 179.05 mmol, 1.4 eq) were added sequentially. The reaction system was stirred at 25°C for 12 hours. LCMS monitored the complete consumption of the starting materials and the formation of the main product. TLC (petroleum ether/ethyl acetate = 10:1) monitored the complete consumption of the starting materials and the formation of a new product spot. 500 mL of water was added to the reaction mixture. The organic phase was separated, and the aqueous phase was extracted with dichloromethane (250 mL * 2). The organic phases were combined, then washed with saturated brine (100 mL * 2), dried over anhydrous sodium sulfate, filtered, and the filtrate was evaporated to dryness under reduced pressure to obtain a residue, which was prepared by silica gel column chromatography (ISCO flash liquid preparative chromatograph; column model: 120 g SepaFlash^{®} Silica Flash Column; mobile phase gradient: 0 to 10% ethyl acetate/petroleum ether; flow rate: 60 mL/min) to obtain intermediate 1-2 (24 g, yield: 73.85%). LCMS (ESI): m/z calculated for C₁₁H₁₃BrNO⁺. [M+H]⁺=254.02, 256.02, found [M+H]⁺= 253.9, 255.9. **¹H NMR** (400 MHz, CDCl₃) δ ppm 7.38 - 7.47 (m, 4H), 7.20 (br s, 1 H), 5.70 (s, 1 H), 2.22 (s, 3H), 1.90 (s, 3H).

### Step 2: Synthesis of intermediate 1-3

Intermediate **1-2** (23 g, 90.51 mmol, 1 eq) was dissolved in 500 mL of dichloromethane, and aluminum trichloride (72.41 g, 543.04 mmol, 6 eq) was added. The reaction system was stirred at 0°C for 1 hour, then slowly warmed to 25°C and stirred for another 1 hour. LCMS monitored the complete consumption of the starting materials and the formation of the main product. The reaction mixture was quenched by adding 1 L of water. The organic phase was separated, and the aqueous phase was extracted with dichloromethane (500 mL * 2). The organic phases were combined, then washed with saturated sodium bicarbonate solution (300 mL * 2), washed with saturated brine (300 mL * 2), dried over anhydrous sodium sulfate, filtered, and the filtrate was evaporated to dryness under reduced pressure to obtain a residue, which was prepared by silica gel column chromatography (ISCO flash liquid preparative chromatograph; column model: 120 g SepaFlash^{®} Silica Flash Column; mobile phase gradient: 0 to 30% ethyl acetate/petroleum ether; flow rate: 30 mL/min) to obtain intermediate **1-3** as a white solid (21 g, yield: 91.30%). LCMS (ESI): m/z calculated for C₁₁H₁₃BrNO⁺. [M+H]⁺= 254.02, 256.02, found [M+H]⁺= 253.9, 255.9. **¹H NMR** (400 MHz, CDCl₃) δ ppm 9.34 (br s, 1 H), 7.40 (d, J=1.98 Hz, 1 H), 7.29 (dd, J=8.36, 2.20 Hz, 1 H), 6.75 (d, J=8.36 Hz, 1 H), 2.48 (s, 2 H), 1.33 (s, 6 H).

### Step 3: Synthesis of intermediate 1-4

Intermediate **1-3** (6 g, 23.61 mmol, 1 eq) was dissolved in 60 mL of toluene, and borane dimethyl sulfide solution (10 M, 3.07 mL, 1.3 eq) was added dropwise to the reaction at 0°C. After the dropwise addition was completed, the reaction system was slowly warmed to room temperature, then further heated to 120°C and refluxed for 5 hours. TLC (petroleum ether:ethyl acetate = 1:1) monitored the complete consumption of the starting materials. LCMS monitored the formation of the main product. The reaction system was quenched with methanol, and saturated sodium bicarbonate solution (200 mL) was added. The mixture was extracted with dichloromethane (500 mL * 2). The organic phases were combined, then washed with saturated brine (100 mL * 2), dried over anhydrous sodium sulfate, filtered, and the filtrate was evaporated to dryness under reduced pressure to obtain a residue, which was prepared by silica gel column chromatography (ISCO flash liquid preparative chromatograph; column model: 40 g SepaFlash^{®} Silica Flash Column; mobile phase gradient: 0 to 2% ethyl acetate/petroleum ether; flow rate: 40 mL/min) to obtain intermediate **1-4** as a light yellow oil (5 g, yield: 88.19%). LCMS (ESI): m/z calculated for C₁₁H₁₅BrN ⁺. [M+H]⁺= 240.04, 242.04, found [M+H]⁺= 239.9, 241.9. **¹H NMR** (400 MHz, CDCl₃) δ ppm 7.25 (d, J=2.20 Hz, 1 H), 7.02 (dd, J=8.58, 2.42 Hz, 1 H), 6.35 (d, J=8.58 Hz, 1 H), 6.35 (d, J=8.58 Hz, 1 H), 3.25 - 3.35 (m, 2 H), 1.66 - 1.76 (m, 2 H), 1.28 (s, 6 H).

### Step 4: Synthesis of intermediate 1-5

Intermediate **1-4** (4.5 g, 18.74 mmol, 1 eq) and sodium bicarbonate (2.72 g, 32.35 mmol, 1.73 eq) were dissolved in 8 mL of water, and dimethyl sulfate (4.82 g, 38.23 mmol, 2.04 eq) was added. The reaction system was stirred at 10°C for 1 hour, then slowly warmed to 50°C and stirred for another 1 hour. The reaction mixture was diluted with 50 mL of water and extracted with dichloromethane (50 mL * 2). The organic phases were combined, then washed with saturated brine (30 mL * 2), dried over anhydrous sodium sulfate, filtered, and the filtrate was evaporated to dryness under reduced pressure to obtain a residue, which was prepared by silica gel column chromatography (ISCO flash liquid preparative chromatograph; column model: 40 g SepaFlash^{®} Silica Flash Column; mobile phase gradient: 0 to 2% ethyl acetate/petroleum ether; flow rate: 40 mL/min) to obtain intermediate **1-5** as a light yellow oil (4.2 g, yield: 88.19%). LCMS (ESI): m/z calculated for C₁₂H₁₇BrN ⁺. [M+H]⁺= 254.05, 256.05, found [M+H]⁺= 253.9, 255.9. **¹H NMR** (400 MHz, CDCl₃) δ ppm 7.24 (d, J=2.38 Hz, 1 H), 7.14 (dd, J=8.76, 2.38 Hz, 1 H), 6.44 (d, J=8.76 Hz, 1 H), 3.19 - 3.26 (m, 2 H), 2.88 (s, 3 H), 1.69 - 1.82 (m, 2 H), 1.27 (s, 6 H).

### Step 5: Synthesis of intermediate 1-6

Intermediate **1-5** (1 g, 3.93 mmol, 1 eq) and compound **1-5a** (1.10 g, 4.33 mmol, 1.1 eq) were dissolved in 10 mL of dioxane, and potassium acetate (772.25 mg, 7.87 mmol, 2 eq) and Pd(dppf)Cl₂ (287.89 mg, 393.44 µmol, 0.1 eq) were added sequentially. The reaction system was stirred at 100°C for 12 hours. TLC (petroleum ether:ethyl acetate = 4:1) monitored the complete consumption of the starting materials and the formation of the main product. The reaction mixture was diluted with 20 mL of ethyl acetate and 20 mL of water. The organic phase was separated, and the aqueous phase was extracted with ethyl acetate (10 mL * 3). The organic phases were combined, then washed with saturated brine (10 mL * 2), dried over anhydrous sodium sulfate, filtered, and the filtrate was evaporated to dryness under reduced pressure to obtain a residue, which was prepared by silica gel column chromatography (ISCO flash liquid preparative chromatograph; column model: 40 g SepaFlash^{®} Silica Flash Column; mobile phase gradient: 0 to 30% ethyl acetate/petroleum ether; flow rate: 60 mL/min) to obtain intermediate 1-6 as a light yellow oil (927 mg, yield: 78.3%). **LCMS** (ESI): m/z calculated for C₁₈H₂₉BNO₂⁺. [M+H]⁺= 302.23, found [M+H]⁺= 302.3.

### Step 6: Synthesis of intermediate 1-7

Intermediate **I-6a** (1.1 g, 2.80 mmol, 1 eq) (preparation method referenced in patent CN113501758A, 2021) and intermediate **I-6** (842.65 mg, 2.80 mmol, 1 eq) were dissolved in 1.5 mL of water and 10 mL of dioxane, and Pd(dppf)Cl₂ (102.34 mg, 139.87 µmol, 0.05 eq) and potassium fluoride (487.55 mg, 8.39 mmol, 3 eq) were added. The reaction system was stirred at 110°C for 1 hour. LCMS monitored the complete consumption of the starting materials and the formation of the main product. The reaction mixture was diluted with 60 mL of ethyl acetate and 20 mL of water. The organic phase was washed with saturated ammonium chloride solution (20 mL * 2), washed with and brine (10 mL), dried over anhydrous sodium sulfate, filtered, and the filtrate was evaporated to dryness under reduced pressure to obtain a residue, which was prepared by silica gel column chromatography (ISCO flash liquid preparative chromatograph; column model: 40 g SepaFlash^{®} Silica Flash Column; mobile phase gradient: 0 to 60% ethyl acetate/petroleum ether; flow rate: 30 mL/min) to obtain intermediate **1-7** as a light yellow oil (1.1 g, yield: 78.39%). **LCMS** (ESI): m/z calculated for C₃₁H₃₆NO₅⁺. [M+H]⁺= 502.26, found [M+H]⁺= 502.2.

### Step 7: Synthesis of compound Y-001

Intermediate **1-7** (1.1 g, 2.19 mmol, 1 eq) was dissolved in 50 mL of methanol, and aqueous sodium hydroxide solution (1 M, 8.77 mL, 4 eq) was added. The reaction system was stirred at 60°C for 3 hours. LCMS monitored the complete consumption of the starting materials and the formation of the main product. The pH value of the reaction system was adjusted to 7 with 1 M hydrochloric acid, and the mixture was extracted with ethyl acetate (50 mL * 2). The organic phases were combined, then washed with brine (30 mL), dried over anhydrous sodium sulfate, filtered, and the filtrate was evaporated to dryness under reduced pressure to obtain a residue, which was prepared by silica gel column chromatography (ISCO flash liquid preparative chromatograph; column model: 40 g SepaFlash^{®} Silica Flash Column; mobile phase gradient: 0 to 70% ethyl acetate/petroleum ether; flow rate: 30 mL/min) to obtain compound **Y-001** as a white solid (470 mg, yield: 46.2%, purity: 93%). **LCMS** (ESI): m/z calculated for C₂₇H₃₀NO₄⁺. [M+H]⁺= 432.22, found [M+H]⁺= 432.2. **¹H NMR** (400 MHz, DMSO-d6) δ ppm 7.98 (d, J=8.3 Hz, 2H), 7.79 (d, J=8.3 Hz, 2H), 7.60 - 7.50 (m, 3H), 7.27 (dd, J=2.0, 8.6 Hz, 1H), 7.16 (d, J=8.7 Hz, 1H), 6.60 (d, J=8.6 Hz, 1H), 4.80 (t, J=5.1 Hz, 1H), 4.14 - 4.01 (m, 2H), 3.73 (q, J=5.0 Hz, 2H), 3.24 - 3.19 (m, 2H), 2.88 (s, 3H), 1.81 - 1.67 (m, 2H), 1.27 (s, 6H).

### Example 2 Synthesis of compound Y-002

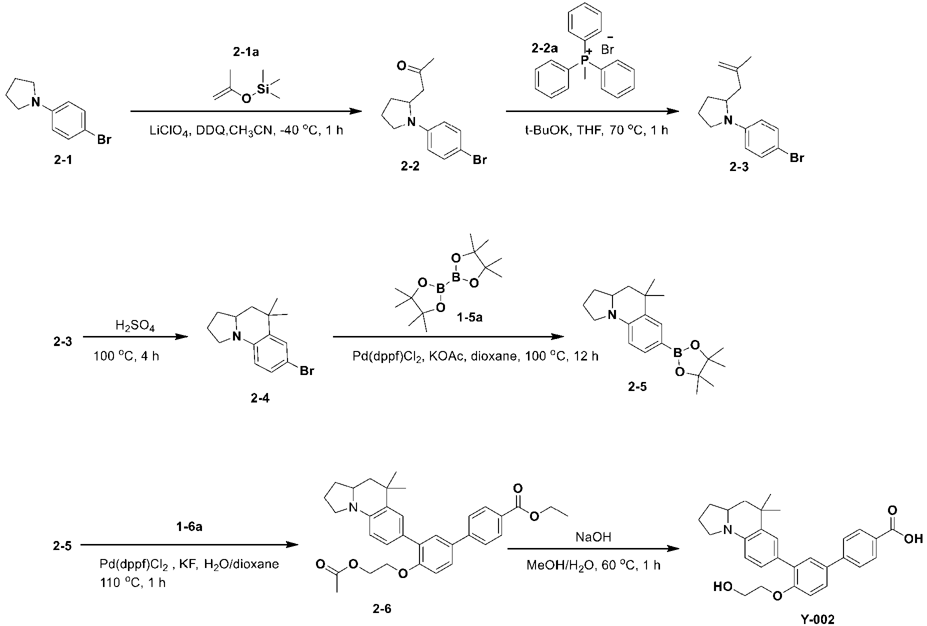

### Step 1 Synthesis of intermediate 2-2

Intermediate **2-1** (7 g, 30.96 mmol, 1 eq) and intermediate **2-1a** (20.16 g, 154.79 mmol, 5 eq) were dissolved in 150 mL of acetonitrile, and lithium perchlorate (6.45 g, 60.63 mmol, 1.96 eq) and 2,3-dichloro-5,6-dicyano-1,4-benzoquinone (DDQ) (21.08 g, 92.87 mmol, 3 eq) were added. The reaction system was stirred at -40°C for 1 hour. TLC (petroleum ether/ethyl acetate = 7:1) monitored the complete reaction of the starting materials and the formation of the main product. The reaction mixture was quenched with saturated aqueous sodium sulfite solution (20 mL), and 30 mL of ethyl acetate and 30 mL of water were added. The organic phase was separated, and the aqueous phase was extracted with ethyl acetate (10 mL * 2). The organic phases were combined, then washed with saturated brine (10 mL * 2), dried over anhydrous sodium sulfate, filtered, and the filtrate was evaporated to dryness under reduced pressure to obtain a residue, which was prepared by silica gel column chromatography (ISCO flash liquid preparative chromatograph; column model: 40 g SepaFlash^{®} Silica Flash Column; mobile phase gradient: 0 to 20% ethyl acetate/petroleum ether; flow rate: 30 mL/min) to obtain intermediate **2-2** as a colorless oil (4.4 g, yield: 50.37%). **LCMS** (ESI): m/z calculated for C₁₃H₁₇BrNO⁺. [M+H]⁺= 282.05, 284.05, found [M+H]⁺= 282.0, 284.0.

### Step 2 Synthesis of intermediate 2-3

Intermediate **2-2** (1.4 g, 4.96 mmol, 1 eq) was dissolved in 50 mL of tetrahydrofuran, and intermediate **2-2a** (3.90 g, 10.92 mmol, 2.2 eq) and potassium tert-butoxide (1.11 g, 9.92 mmol, 2 eq) were added. The reaction system was stirred at 70°C for 1 hour. TLC (petroleum ether/ethyl acetate = 10:1) monitored the complete reaction of the starting materials. LCMS monitored the formation of the product. The reaction mixture was concentrated under reduced pressure to obtain a residue, which was prepared by silica gel column chromatography (ISCO flash liquid preparative chromatograph; column model: 40 g SepaFlash^{®} Silica Flash Column; mobile phase gradient: 0 to 30% ethyl acetate/petroleum ether; flow rate: 60 mL/min) to obtain intermediate **2-3** as a colorless oil (1.1 g, yield: 79.14%). **LCMS** (ESI): m/z calculated for C₁₄H₁₉BrN⁺. [M+H]⁺= 280.07, 282.07, found [M+H]⁺= 280.1, 282.1. **¹H NMR** (400 MHz, CDCl₃) δ ppm 7.34 - 7.29 (m, 2H), 6.47 (d, J=9.0 Hz, 2H), 4.86 (s, 1H), 4.77 (s, 1H), 3.95 - 3.77 (m, 1H), 3.43 - 3.35 (m, 1H), 3.19 - 3.10 (m, 1H), 2.44 (br d, J=14.3 Hz, 1H), 2.13 - 1.89 (m, 5H), 1.84 (s, 3H).

### Step 3 Synthesis of intermediate 2-4

Intermediate **2-3** (1 g, 3.57 mmol, 1 eq) was dissolved in 10 mL of concentrated sulfuric acid, and the reaction system was stirred at 100°C for 4 hours. LCMS monitored the formation of the product. The reaction mixture was slowly added dropwise to saturated sodium bicarbonate solution at 0°C. After the dropwise addition was completed, the aqueous phase was extracted with ethyl acetate (30 mL * 3). The organic phases were combined, then washed with saturated brine (30 mL * 2), dried over anhydrous sodium sulfate, filtered, and the filtrate was evaporated to dryness under reduced pressure to obtain a residue, which was prepared by silica gel column chromatography (ISCO flash liquid preparative chromatograph; column model: 20 g SepaFlash^{®} Silica Flash Column; mobile phase gradient: 0 to 40% ethyl acetate/petroleum ether; flow rate: 60 mL/min) to obtain intermediate **2-4** as a white solid (870 mg, yield: 87%). **LCMS** (ESI): m/z calculated for C₁₄H₁₉BrN⁺. [M+H]⁺= 280.07, 282.07, found [M+H]⁺= 280.0, 282.0. **¹H NMR** (400 MHz, CDCl₃) δ ppm 7.25 (d, J=2.4 Hz, 1H), 7.13 (dd, J=2.4, 8.7 Hz, 1H), 6.27 (d, J=8.6 Hz, 1H), 3.54 - 3.41 (m, 1H), 3.36 - 3.27 (m, 1H), 3.25 - 3.15 (m, 1H), 2.18 - 2.04 (m, 2H), 2.00 - 1.89 (m, 1H), 1.80 (dd, J=3.3, 12.7 Hz, 1H), 1.49 - 1.42 (m, 1H), 1.35 (s, 4H), 1.21 (s, 3H).

### Step 4 Synthesis of intermediate 2-5

Intermediate **2-4** (110 mg, 392.57 µmol, 1 eq) and compound **1-5a** (109.66 mg, 431.83 µmol, 1.1 eq) were dissolved in 15 mL of dioxane, and potassium acetate (77.05 mg, 785.15 µmol, 2 eq) and Pd(dppf)Cl₂ (28.72 mg, 39.26 µmol, 0.1 eq) were added sequentially. The reaction system was stirred at 100°C for 12 hours. TLC (petroleum ether:ethyl acetate = 4:1) monitored the complete consumption of the starting materials and the formation of the main product. The reaction mixture was diluted with 20 mL of ethyl acetate and 20 mL of water. The organic phase was separated, and the aqueous phase was extracted with ethyl acetate (10 mL * 3). The organic phases were combined, then washed with saturated brine (10 mL * 2), dried over anhydrous sodium sulfate, filtered, and the filtrate was evaporated to dryness under reduced pressure to obtain a residue, which was prepared by silica gel column chromatography (ISCO flash liquid preparative chromatograph; column model: 40 g SepaFlash^{®} Silica Flash Column; mobile phase gradient: 0 to 30% ethyl acetate/petroleum ether; flow rate: 60 mL/min) to obtain intermediate **2-5** as a colorless oil (70 mg, yield: 54.48%). **LCMS** (ESI): m/z calculated for C₂₀H₃₁BNO₂⁺. [M+H]⁺= 328.24, found [M+H]⁺= 328.2. **¹H NMR** (400 MHz, CDCl₃) δ ppm 7.64 (s, 1H), 7.55 (d, J=8.2 Hz, 1H), 6.40 (d, J=8.1 Hz, 1H), 3.58 - 3.49 (m, 1H), 3.43 - 3.35 (m, 1H), 3.32 -3.24 (m, 1H), 2.17 - 2.05 (m, 2H), 2.02 - 1.89 (m, 1H), 1.81 (dd, J=3.3, 12.6 Hz, 1H), 1.49 - 1.41 (m, 4H), 1.32 (s, 13H), 1.22 (s, 3H).

### Step 5 Synthesis of intermediate 2-6

Intermediate **1-6a** (79 mg, 200.90 µmol, 1 eq) and intermediate **2-5** (65.75 mg, 200.90 µmol, 1 eq) were dissolved in 1 mL of water and 4 mL of dioxane, and Pd(dppf)Cl₂ (7.35 mg, 10.05 µmol, 0.05 eq) and potassium fluoride (35.02 mg, 602.70 µmol, 3 eq) were added. The reaction system was stirred at 110°C for 1 hour. LCMS monitored the complete consumption of the starting materials and the formation of the main product. The reaction mixture was diluted with 15 mL of ethyl acetate and 10 mL of water. The organic phase was washed with saturated ammonium chloride solution (10 mL * 2), washed with brine (8 mL), dried over anhydrous sodium sulfate, filtered, and the filtrate was evaporated to dryness under reduced pressure to obtain a residue, which was prepared by silica gel column chromatography (ISCO flash liquid preparative chromatograph; column model: 40 g SepaFlash^{®} Silica Flash Column; mobile phase gradient: 0 to 60% ethyl acetate/petroleum ether; flow rate: 30 mL/min) to obtain intermediate **2-6** as a colorless oil (80 mg, yield: 75.47%). **LCMS** (ESI): m/z calculated for C₃₃H₃₈NO₅⁺. [M+H]⁺= 528.27, found [M+H]⁺= 528.2.

### Step 6 Synthesis of compound Y-002

Intermediate **2-6** (80 mg, 151.62 µmol, 1 eq) was dissolved in 4 mL of methanol, and sodium hydroxide solution (1 M, 758.08 µL, 4 eq) was added. The reaction system was stirred at 60°C for 1 hour. LCMS monitored the complete consumption of the starting materials and the formation of the main product. The pH value of the reaction system was adjusted to 7 with 1 M hydrochloric acid, and the mixture was extracted with ethyl acetate (10 mL * 2). The organic phases were combined, then washed with brine (10 mL), dried over anhydrous sodium sulfate, filtered, and the filtrate was evaporated to dryness under reduced pressure to obtain a residue, which was prepared by silica gel column chromatography (ISCO flash liquid preparative chromatograph; column model: 12 g SepaFlash^{®} Silica Flash Column; mobile phase gradient: 0 to 70% ethyl acetate/petroleum ether; flow rate: 30 mL/min) to obtain compound **Y-002** as a white solid (42 mg, yield: 58.16%, purity: 96%). **LCMS** (ESI): m/z calculated for C₂₉H₃₂NO₄⁺. [M+H]⁺= 458.23, found [M+H]⁺= 458.2. **¹H NMR** (400 MHz, DMSO-d6) δ ppm 7.99 (d, J=8.3 Hz, 2H), 7.79 (d, J=8.2 Hz, 2H), 7.61 - 7.49 (m, 3H), 7.27 (dd, J=1.9, 8.3 Hz, 1H), 7.19 - 7.11 (m, 1H), 6.41 (d, J=8.5 Hz, 1H), 4.79 (t, J=5.1 Hz, 1H), 4.07 (t, J=5.2 Hz, 2H), 3.73 (q, J=5.1 Hz, 2H), 3.46 (br d, J=10.7 Hz, 2H), 3.26 - 3.16 (m, 1H), 2.17 - 2.00 (m, 2H), 1.97 - 1.80 (m, 2H), 1.51 - 1.39 (m, 1H), 1.38 - 1.29 (m, 4H), 1.21 (s, 3H).

### Example 3 Synthesis of compound Y-003

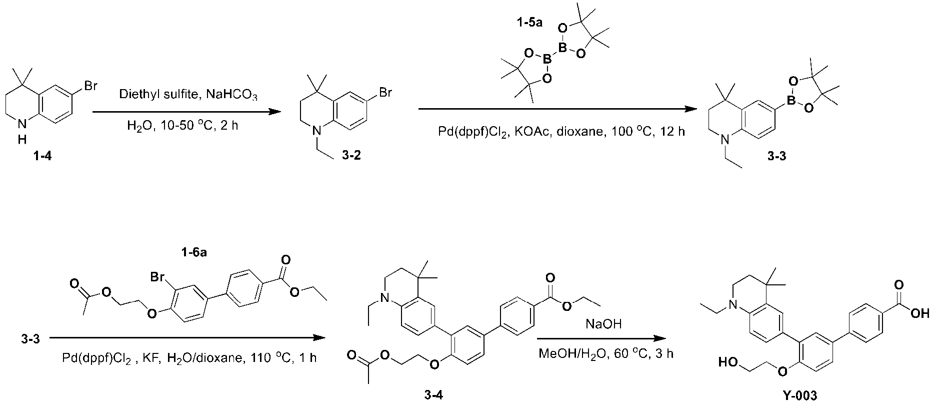

### Step 1 Synthesis of intermediate 3-2

Referring to the synthesis method in Step 4 of **Example 1,** intermediate **3-2** was prepared as a colorless oil using intermediate **1-4** and diethyl sulfate as starting materials, with the corresponding starting materials being replaced. **LCMS** (ESI): m/z calculated for C₁₃H₁₉BrN⁺. [M+H]⁺= 268.07, 270.07, found [M+H]⁺= 268.0, 270.0. **¹H NMR** (400 MHz, CDCl₃) δ ppm 7.25 (d, J=2.42 Hz, 1 H), 7.12 (dd, J=8.80, 2.42 Hz, 1 H), 6.48 (d, J=8.80 Hz, 1 H), 3.35 (q, J=7.12 Hz, 2 H), 3.24 - 3.29 (m, 2 H), 1.69 - 1.78 (m, 2 H), 1.27 (s, 6 H), 1.15 (t, J=7.04 Hz, 3 H).

### Step 2 Synthesis of intermediate 3-3

Referring to the synthesis method in Step 5 of **Example 1**, intermediate **3-3** was prepared as a colorless oil using intermediate **3-2** as a starting material, with the corresponding starting materials being replaced. **LCMS** (ESI): m/z calculated for C₁₉H₃₁BNO₂ ⁺. [M+H]⁺= 316.24, found [M+H]⁺= 316.2. **¹H NMR** (400 MHz, CDCl₃) δ ppm 7.62 (d, J=1.2 Hz, 1H), 7.52 (dd, J=1.4, 8.2 Hz, 1H), 6.59 (d, J=8.2 Hz, 1H), 3.39 (q, J=7.0 Hz, 2H), 3.34 - 3.28 (m, 2H), 1.75 - 1.70 (m, 2H), 1.32 (s, 12H), 1.30 (s, 6H), 1.15 (t, J=7.0 Hz, 3H).

### Step 3 Synthesis of compound Y-003

Referring to the synthesis methods in Step 6 and Step 7 of **Example 1**, compound **Y-003** was prepared as a white solid using intermediate **3-3** as a starting material, with the corresponding starting materials being replaced. **LCMS** (ESI): m/z calculated for C₂₈H₃₂NO₄ ⁺. [M+H]⁺= 446.23, found [M+H]⁺= 446.2. **¹H NMR** (400 MHz, DMSO-d6) δ ppm 7.99 (d, J=8.3 Hz, 2H), 7.80 (d, J=8.5 Hz, 2H), 7.60 - 7.52 (m, 3H), 7.26 (dd, J=2.1, 8.5 Hz, 1H), 7.19 - 7.11 (m, 1H), 6.63 (d, J=8.7 Hz, 1H), 4.82 (br s, 1H), 4.14 - 4.06 (m, 2H), 3.75 (br s, 2H), 3.41 - 3.39 (m, 2H), 3.30 - 3.24 (m, 2H), 1.76 - 1.66 (m, 2H), 1.26 (s, 6H), 1.09 (t, J=7.0 Hz, 3H).

### Example 4 Chiral separation and preparation of compounds Y-004 and Y-005

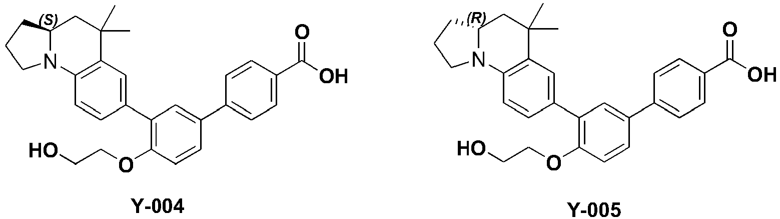

Compound **Y-002** (22 mg) prepared in **Example 2** was further separated by chiral SFC under chiral preparative conditions: chiral column DAICEL CHIRALPAK AD (specification: 250 mm * 30 mm, particle size: 10 µm), eluent phase: CO₂ (A): ethanol containing 0.1% aqueous ammonia (B), isocratic gradient (A/B = 60/40). Compound **Y-004** (earlier-eluting peak) was obtained as a white solid (8.2 mg, yield: 36.52%, ee value: 98%).

**LCMS (ESI):** m/z calculated for C₂₉H₃₂NO₄⁺. [M+H]⁺= 458.23, found [M+H]⁺= 458.2.

**¹H NMR** (400 MHz, CD₃OD) δ ppm 7.96 (d, J=8.3 Hz, 2H), 7.60 (d, J=8.3 Hz, 2H), 7.49 - 7.38 (m, 3H), 7.16 (dd, J=1.9, 8.3 Hz, 1H), 7.03 (d, J=8.5 Hz, 1H), 6.38 (d, J=8.5 Hz, 1H), 3.99 (t, J=4.9 Hz, 2H), 3.76 (t, J=4.9 Hz, 2H), 3.49 - 3.36 (m, 1H), 3.31 - 3.23 (m, 2H), 2.10 - 1.98 (m, 2H), 1.95 - 1.83 (m, 1H), 1.77 (dd, J=3.1, 12.6 Hz, 1H), 1.41-1.38 (m, 1H), 1.31 - 1.28 (m, 4H), 1.17 (s, 3H).

Compound **Y-005** (later-eluting peak) was obtained as a white solid (7.9 mg, yield: 35.19%, ee value: 98%).

**LCMS (ESI):** m/z calculated for C₂₉H₃₂NO₄⁺. [M+H]⁺= 458.23, found [M+H]⁺= 458.2.

**¹H NMR** (400 MHz, CD₃OD) δ ppm 8.09 (d, J=8.3 Hz, 2H), 7.73 (d, J=8.3 Hz, 2H), 7.60 - 7.49 (m, 3H), 7.32 - 7.24 (m, 1H), 7.15 (d, J=8.5 Hz, 1H), 6.50 (d, J=8.3 Hz, 1H), 4.11 (t, J=4.9 Hz, 2H), 3.94 - 3.82 (m, 2H), 3.59 - 3.48 (m, 1H), 3.42 - 3.35 (m, 2H), 2.21 - 2.13 (m, 2H), 2.08 - 1.95 (m, 1H), 1.89 (dd, J=3.0, 12.6 Hz, 1H), 1.56 - 1.46 (m, 1H), 1.44 - 1.40 (m, 4H), 1.29 (s, 3H).

Chiral analysis conditions: Instrument Waters UPCC equipped with PDA detector, chiral column Chiralpak AD-3 (specification: 150 mm * 4.6 mm, particle size: 3 µm), eluent phase: CO₂ (A): ethanol containing 0.05% ethylenediamine (B), isocratic gradient (A/B = 60/40); flow rate: 2.5 mL/min, column temperature: 35°C; compound **Y-004,** earlier-eluting peak, retention time: about 4.53 min; compound **Y-005,** later-eluting peak, retention time: about 5.45 min (the absolute chirality of the separated compound was confirmed by comparing the retention time of the separated compound with the retention time of the product **Y-005** obtained from chiral preparation in Example 5).

### Example 5 Synthesis of compound Y-005

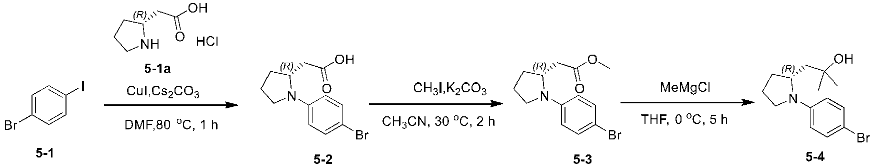

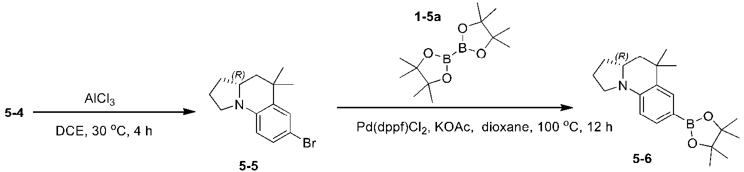

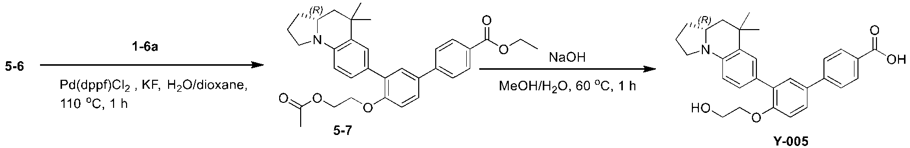

### Step 1 Synthesis of intermediate 5-2

Compound **5-1** (2 g, 7.07 mmol, 1 eq) was dissolved in 3 mL of DMF, and cesium carbonate (5.76 g, 17.67 mmol, 2.5 eq), cuprous iodide (403.92 mg, 2.12 mmol, 0.3 eq), and compound 5-1a (1.19 g, 7.07 mmol, 1 eq) were added sequentially. The reaction mixture was stirred at 80°C for 1 hour. LCMS monitored the complete consumption of the starting materials and the formation of the main product. After filtration of the reaction system, the filtrate was diluted with 20 mL of ethyl acetate, washed with saturated ammonium chloride solution (10 mL * 2), washed with saturated brine (10 mL * 2), dried over anhydrous sodium sulfate, filtered, and the filtrate was evaporated to dryness under reduced pressure to obtain a residue, which was prepared by silica gel column chromatography (ISCO flash liquid preparative chromatograph; column model: 40 g SepaFlash^{®} Silica Flash Column; mobile phase gradient: 0 to 70% ethyl acetate/petroleum ether; flow rate: 30 mL/min) to obtain intermediate **5-2** as a colorless oil (1.3 g, yield: 64.71%). **LCMS** (ESI): m/z calculated for C₁₂H₁₅BrNO₂⁺. [M+H]⁺= 284.03, 286.03, found [M+H]⁺= 284.0, 286.0.

### Step 2 Synthesis of intermediate 5-3

Compound **5-2** (1.3 g, 4.58 mmol, 1 eq) was dissolved in 40 mL of acetonitrile, and potassium carbonate (1.90 g, 13.73 mmol, 3 eq) and iodomethane (6.49 g, 45.75 mmol, 2.85 mL, 10 eq) were added. The reaction system was stirred at 30°C for 2 hours. TLC (petroleum ether:ethyl acetate = 5:1) monitored the complete consumption of the starting materials and the formation of the main product spot. The reaction mixture was filtered, and the filtrate was concentrated under reduced pressure to obtain a residue, which was prepared by silica gel column chromatography (ISCO flash liquid preparative chromatograph; column model: 40 g SepaFlash^{®} Silica Flash Column; mobile phase gradient: 0 to 12% ethyl acetate/petroleum ether; flow rate: 60 mL/min) to obtain intermediate **5-3** as a colorless oil (900 mg, yield: 65.97%). **LCMS** (ESI): m/z calculated for C₁₃H₁₇BrNO₂⁺. [M+H]⁺= 298.04, 300.04, found [M+H]⁺= 298.0, 300.0. **¹H NMR** (400 MHz, CD₃OD) δ ppm 7.35 - 7.29 (m, 2H), 6.49 (d, J=8.9 Hz, 2H), 4.15 (dd, J=3.5, 6.5 Hz, 1H), 3.73 (s, 3H), 3.41 - 3.38 (m, 1H), 3.16 (d, J=8.2 Hz, 1H), 2.78 - 2.73 (m, 1H), 2.27 - 2.22 (m, 1H), 2.09 - 2.06 (m, 3H), 1.91 (dd, J=2.9, 6.7 Hz, 1H).

### Step 3 Synthesis of intermediate 5-4

Compound **5-3** (1.30 g, 4.36 mmol, 1 eq) was dissolved in 10 mL of tetrahydrofuran, and a solution of methylmagnesium chloride in tetrahydrofuran (3 M, 5.81 mL, 4 eq) was slowly added dropwise at 0°C. After the dropwise addition was completed, the reaction system was stirred at 0°C for 5 hours. LCMS monitored the complete consumption of the starting materials and the formation of the product. The reaction mixture was diluted with 20 mL of water and 10 mL of ethyl acetate. The organic phase was separated, and the aqueous phase was extracted with ethyl acetate (10 mL * 2). The organic phases were combined, then dried over anhydrous sodium sulfate, filtered, and the filtrate was evaporated to dryness under reduced pressure to obtain a residue, which was prepared by silica gel column chromatography (ISCO flash liquid preparative chromatograph; column model: 12 g SepaFlash^{®} Silica Flash Column; mobile phase gradient: 0 to 56% ethyl acetate/petroleum ether; flow rate: 30 mL/min) to obtain intermediate **5-4** as a colorless oil (550 mg, yield: 42.30%). **LCMS** (ESI): m/z calculated for C₁₄H₂₁BrNO⁺. [M+H]⁺= 298.08, 300.08, found [M+H]⁺= 298.1, 300.1.

### Step 4 Synthesis of intermediate 5-5

Compound **5-4** (550 mg, 1.84 mmol, 1 eq) was dissolved in 10 mL of dichloroethane, and aluminum trichloride (491.83 mg, 3.69 mmol, 2 eq) was added. The reaction system was stirred at 30°C for 4 hours. LCMS monitored the complete consumption of the starting materials and the formation of the product. The reaction mixture was slowly added dropwise to 15 mL of saturated aqueous sodium carbonate solution. The organic phase was separated, and the aqueous phase was extracted with ethyl acetate (5 mL * 2). The organic phases were combined, then dried over anhydrous sodium sulfate, filtered, and the filtrate was evaporated to dryness under reduced pressure to obtain a residue, which was prepared by silica gel column chromatography (ISCO flash liquid preparative chromatograph; column model: 4 g SepaFlash^{®} Silica Flash Column; mobile phase gradient: 0 to 3% ethyl acetate/petroleum ether; flow rate: 20 mL/min) to obtain intermediate **5-5** as a colorless oil (140 mg, yield: 27.15%). LCMS (ESI): m/z calculated for C₁₄H₁₉BrN⁺. [M+H]⁺= 280.07, 282.07, found [M+H]⁺= 280.0, 282.0.

### Step 5 Synthesis of compound Y-005

Referring to the synthesis methods in Steps **4-6** of **Example 2**, compound **Y-005** was prepared as a white solid using intermediate **5-5** as a starting material, with the corresponding starting materials being replaced. **LCMS (ESI):** m/z calculated for C₂₉H₃₂NO₄⁺. [M+H]⁺= 458.23, found [M+H]⁺= 458.2. Chiral analysis conditions: Instrument Waters UPCC equipped with PDA detector, chiral column Chiralpak AD-3 (specification: 150 mm * 4.6 mm, particle size: 3 µm), eluent phase: CO₂ (A): ethanol containing 0.05% ethylenediamine (B), isocratic gradient (A/B = 60/40); flow rate: 2.5 mL/min, column temperature: 35°C; retention time: about 5.45 minutes.

### Example 6 Synthesis of compound Y-006

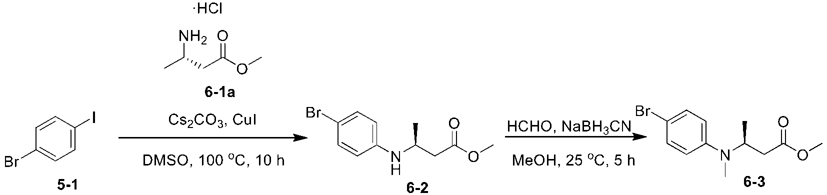

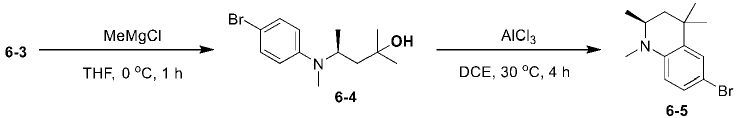

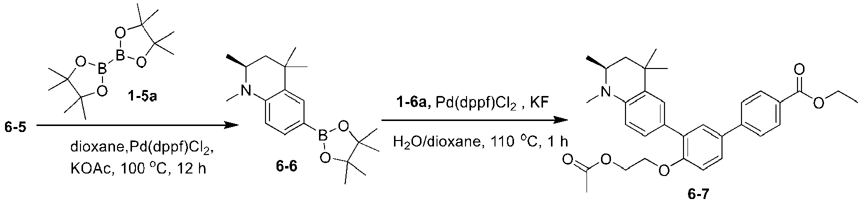

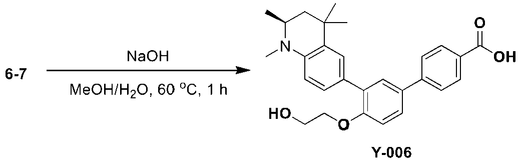

### Step 1 Synthesis of intermediate 6-2

Intermediate 5-1 (2.98 g, 10.55 mmol, 2 eq) and compound **6-1a** (810 mg, 5.27 mmol, 1 eq) were dissolved in 10 mL of DMSO, and cesium carbonate (4.30 g, 13.18 mmol, 2.5 eq) and cuprous iodide (301.28 mg, 1.58 mmol, 0.3 eq) were added sequentially. The reaction system was stirred at 100°C for 10 hours. LCMS monitored the complete consumption of the starting materials and the formation of the main product. After filtration of the reaction system, the filtrate was diluted with 30 mL of ethyl acetate, washed with saturated ammonium chloride solution (10 mL * 2), washed with saturated brine (10 mL * 2), dried over anhydrous sodium sulfate, filtered, and the filtrate was evaporated to dryness under reduced pressure to obtain a residue, which was prepared by silica gel column chromatography (ISCO flash liquid preparative chromatograph; column model: 40 g SepaFlash^{®} Silica Flash Column; mobile phase gradient: 0 to 80% ethyl acetate/petroleum ether; flow rate: 20 mL/min) to obtain intermediate **6-2** as a colorless oil (1.15 g, yield: 80.18%). **LCMS** (ESI): m/z calculated for C₁₁H₁₅BrNO₂⁺. [M+H]⁺= 272.03, 274.03, found [M+H]⁺= 272.0, 274.0.

### Step 2 Synthesis of intermediate 6-3

Intermediate **6-2** (400 mg, 1.47 mmol, 1 eq) was dissolved in 5 mL of methanol, and 30% aqueous formaldehyde solution (735.56 mg, 7.35 mmol, 674.82 µL, 5 eq) and sodium cyanoborohydride (461.84 mg, 7.35 mmol, 476.12 µL, 5 eq) were added sequentially. The reaction system was stirred at 25°C for 5 hours. LCMS monitored the complete consumption of the starting materials and the formation of the main product. The reaction mixture was concentrated under reduced pressure to obtain a residue, which was prepared by silica gel column chromatography (ISCO flash liquid preparative chromatograph; column model: 12 g SepaFlash^{®} Silica Flash Column; mobile phase gradient: 0 to 100% ethyl acetate/petroleum ether; flow rate: 30 mL/min) to obtain intermediate 6-3 as a colorless oil (385 mg, yield: 91.52%). **LCMS** (ESI): m/z calculated for C₁₂H₁₇BrNO₂⁺. [M+H]⁺= 286.04, 288.04, found [M+H]⁺= 286.0, 288.0.

### Step 3 Synthesis of intermediate 6-4

Intermediate 6-3 (240 mg, 838.68 µmol, 1 eq) was dissolved in 40 mL of tetrahydrofuran, and a solution of methylmagnesium chloride in tetrahydrofuran (3 M, 1.12 mL, 4 eq) was added dropwise. After the dropwise addition was completed, the reaction system was stirred at 0°C for 1 hour. LCMS monitored the complete consumption of the starting materials and the formation of the product. The reaction mixture was diluted with 10 mL of water and 10 mL of ethyl acetate. The organic phase was separated, and the aqueous phase was extracted with ethyl acetate (10 mL * 2). The organic phases were combined, then dried over anhydrous sodium sulfate, filtered, and the filtrate was evaporated to dryness under reduced pressure to obtain a residue, which was prepared by silica gel column chromatography (ISCO flash liquid preparative chromatograph; column model: 12 g SepaFlash^{®} Silica Flash Column; mobile phase gradient: 0 to 30% ethyl acetate/petroleum ether; flow rate: 30 mL/min) to obtain intermediate **6-4** as a colorless oil (200 mg, yield: 83.38%). **LCMS** (ESI): m/z calculated for C₁₃H₂₁BrNO ⁺. [M+H]⁺= 286.08, 288.08, found [M+H]⁺= 286.0, 288.0.

### Step 4 Synthesis of intermediate 6-5

Compound 6-4 (200 mg, 698.79 µmol, 1 eq) was dissolved in 5 mL of dichloroethane, and aluminum trichloride (93.18 mg, 698.79 µmol, 1 eq) was added. The reaction system was stirred at 30°C for 4 hours. LCMS monitored the complete consumption of the starting materials and the formation of the product. The reaction mixture was slowly added dropwise to 10 mL of saturated aqueous sodium carbonate solution. The organic phase was separated, and the aqueous phase was extracted with ethyl acetate (10 mL * 2). The organic phases were combined, then dried over anhydrous sodium sulfate, filtered, and the filtrate was evaporated to dryness under reduced pressure to obtain a residue, which was prepared by silica gel column chromatography (ISCO flash liquid preparative chromatograph; column model: 4 g SepaFlash^{®} Silica Flash Column; mobile phase gradient: 0 to 5% ethyl acetate/petroleum ether; flow rate: 20 mL/min) to obtain intermediate **6-5** as a colorless oil (160 mg, yield: 85.37%). LCMS (ESI): m/z calculated for C₁₃H₁₉BrN⁺. [M+H]⁺= 268.07, 270.07, found [M+H]⁺= 268.0, 270.0. **¹H NMR** (400 MHz, CD₃OD) δ ppm 7.21 (d, J = 2.3 Hz, 1H), 7.14 (dd, J = 2.4, 8.7 Hz, 1H), 6.46 (br d, J = 8.8 Hz, 1H), 3.40-3.36 (m, 1H), 2.88 (s, 3H), 1.74-1.70 (m, 1H), 1.61-1.58 (m, 1H), 1.32 (s, 3H), 1.24 (br d, J = 6.0 Hz, 3H), 1.18 (s, 3H).

### Step 5 Synthesis of intermediate 6-6

Referring to the synthesis method in Step **5** of **Example 5**, intermediate **6-6** was prepared as a colorless oil using intermediate **6-5** as a starting material, with the corresponding starting materials being replaced. **LCMS (ESI):** m/z calculated for C₁₉H₃₁BNO₂⁺. [M+H]⁺= 316.24, found [M+H]⁺= 316.2. **¹H NMR** (400 MHz, CD₃OD) δ ppm 7.63 - 7.50 (m, 2H), 6.58 (d, J = 8.2 Hz, 1H), 3.54 - 3.33 (m, 1H), 2.94 (s, 3H), 1.77 - 1.68 (m, 1H), 1.62 (s, 1H), 1.39 (s, 3H), 1.32 (s, 12H), 1.25 (d, J = 6.1 Hz, 3H), 1.19 (s, 3H).

### Step 5 Synthesis of compound Y-006

Referring to the synthesis method of Y-005 in **Example 5**, compound **Y-006** was prepared as a white solid using intermediate **6-6** as a starting material, with the corresponding starting materials being replaced. **LCMS (ESI):** m/z calculated for C₂₈H₃₂NO₄⁺. [M+H]⁺= 446.23, found [M+H]⁺= 446.2. **¹H NMR** (400 MHz, CD₃OD) δ ppm 12.90 (br s, 1H), 7.99 (d, J = 8.3 Hz, 2H), 7.80 (d, J = 8.5 Hz, 2H), 7.61 - 7.54 (m, 2H), 7.49 (d, J = 2.0 Hz, 1H), 7.28 (dd, J = 2.0, 8.6 Hz, 1H), 7.16 (d, J = 9.3 Hz, 1H), 6.63 (d, J = 8.7 Hz, 1H), 4.80 (t, J = 5.3 Hz, 1H), 4.07 (t, J = 5.2 Hz, 2H), 3.73 (br d, J = 5.2 Hz, 2H), 3.39 (br s, 1H), 2.88 (s, 3H), 1.76 (dd, J = 4.2, 13.2 Hz, 1H), 1.55 (dd, J = 11.0, 13.1 Hz, 1H), 1.32 (s, 3H), 1.23 (d, J = 6.1 Hz, 3H), 1.19 (s, 3H).

### Biological test example 1

### Using HEK-293 cell lines transfected with RARα, RARβ, and RARγ luciferase reporter genes to test the agonistic activity and selectivity of compounds against RARα, RARβ and RARγ

### Experimental steps:

### 1. Cell culture

HEK293 cells were cultured in DMEM + 10% FBS medium in a humidified CO₂ incubator at 37°C with 5% CO₂.

### 2. Plasmid transfection and cell seeding

HEK293 cells were digested with trypsin, and the HEK293 cell resuspension was adjusted to 200,000 cells per mL with culture medium (96-well plate: 100 µL/well). According to the table below, the transfection reagent mixture was prepared, mixed thoroughly, and allowed to stand at room temperature for 20 minutes.

### Test transfection materials

| **Experimental transfection materials** | **Volume (µL)** |
|---|---|
| pG5 Luc plasmid (100 ng/µL) | 25 |
| pBIND-RARα/ pBIND-RARβ/ pBIND-RARγ (100 ng/µL) * | 25 |
| Opti-MEM medium | 250.0 |
| P3000 | 10.00 |
| Opti-MEM medium | 250.0 |
| Lipofectamine | 7.5 |

| | |
|---|---|
| * The corresponding plasmid (pBIND-RARα, pBIND-RARβ, or pBIND-RARγ) was selected according to the test receptor | |

The prepared transfection reagent mixture was added to 10 mL of the resuspension with adjusted cell density, mixed thoroughly by inverting up and down, and seeded into a 96-well plate at a volume of 100 µL/well. The 96-well plate was placed in a humidified incubator at 37°C with 5% CO₂ and incubated for 24 hours.

### 3. Drug treatment

The stock solutions of the positive control retinoic acid and the test compounds were serially diluted at a certain ratio to obtain a series of 21× compound solutions. To each well of cells (already containing 100 µL of cell culture medium), 5 µL of the retinoic acid dilution or the test compound dilution was added. 5 µL of 10% DMSO medium was added to the negative control wells (Min wells), and 5 µL of 6.3 µM retinoic acid (dissolved in medium, 10% DMSO, final concentration of 300 nM as Max wells) was added to the positive control wells. The assay plate was returned to the incubator and incubated for 24 hours.

### 4. Luciferase assay

The assay plate was detected using Promega's Dual-Glo Luciferase Assay System kit, and the Firefly luciferase fluorescence signal and the Renilla luciferase fluorescence signal were read using an EnSpire microplate reader.

### 5. Test results

### 5.1 EC₅₀ values

The data were imported into MS Excel for curve fitting to obtain EC₅₀ values. The test results are as follows.

### Agonistic activity EC₅₀ test results of RAR series compounds against RARγ, RARα, and RARβ

| Compound No. | **RAR α EC₅₀ (nM)** | **RAR β EC₅₀ (nM)** | **RAR γ EC₅₀ (nM)** | **α/γ ratio** | **β/γ ratio** |
|---|---|---|---|---|---|
| Retinoic acid | 11.46 | 4.84 | 2.65 | 4.3 | 1.8 |
| Y-001 | >6000 | >6000 | 0.37 | >16216.2 | >16216.2 |
| Y-003 | >6000 | >6000 | 2.27 | >2643.2 | >2643.2 |
| Y-004 | >6000 | >6000 | 15.04 | >398.9 | >398.9 |
| Y-005 | >6000 | >6000 | 18.00 | >333.3 | >333.3 |
| Y-006 | >6000 | >6000 | 4.82 | >1244.8 | >1244.8 |

| | | | | | |
|---|---|---|---|---|---|
| Note: When the maximum activation rate is less than 50% of the retinoic acid Max well, the result is expressed as EC₅₀ > maximum test concentration. | | | | | |

The compounds of the present disclosure have good agonistic activity and selectivity against RARγ and almost no agonistic activity against RARα and/or RARβ, with low systemic or other tissue side effects. Compared with existing RARγ selective agonists (for example, compound 10Y), the compounds of the present disclosure have weaker agonistic activity against RARα and RARβ and better selectivity.

### 5.2 Eₘₐₓ values

The activation rate at each concentration was calculated using Formula (1), and the maximum agonistic activity Eₘₐₓ (%) of the compound against RARγ was obtained by curve fitting.$Activation rate \left(%\right) = \frac{final measured value - mean Min value}{mean Max value - mean Min value} \times 100 %$ Formula (1)

The compounds Y-004, Y-005, and Y-006 of the present disclosure exhibit partial agonistic activity against RARγ (Eₘₐₓ values are all less than 75% relative to retinoic acid). Compared with existing RARγ full agonists (for example, retinoic acid, compound 10Y, etc.), these compounds are expected to reduce the adverse reactions associated with full activation of RARγ.

### Biological test example 2

### Study on skin irritation of topical skin administration in miniature pigs

### Experimental design:

The local skin irritation of the test articles was evaluated using the intra-animal self-comparison method in miniature pigs. The dorsal skin of three female miniature pigs was divided into two regions, anterior and posterior, with an administration area of approximately 300 cm² for each region. Four groups of test articles were evaluated crosswise over 4 cycles, with one test article administered to the skin once daily for 3 consecutive days. To avoid unexpected interference, a 2-day washout period was set between the administrations of groups G2 and G3 and between the administrations of groups G3 and G4. The test articles were: G1 blank cream group, G2 Y-004 cream 0.05% group (containing 500 µg of the active ingredient compound Y-004 of the present disclosure per gram of cream, i.e., specification 500 µg/g), G3 10Y cream 0.05% group (containing 500 µg of the active ingredient compound 10Y per gram of cream, i.e., specification 500 µg/g), and G4 10Y cream 0.025% group (containing 250 µg of the active ingredient compound 10Y per gram of cream, i.e., specification 250 µg/g). The cream compositions of the groups G2, G3, and G4 were the same as the blank cream group except for the active ingredient. The structure of compound 10Y is as follows

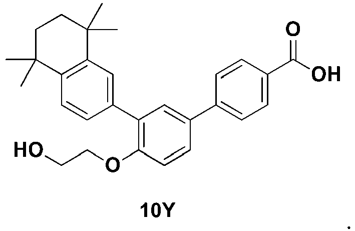

| Administration phase | Cycle 1 (D1-D3) | Cycle 2 (D4-D6) | Cycle 3 (D9-D11) | Cycle 4 (D14-D16) |
|---|---|---|---|---|
| Administration group | G1 (blank cream) | G2 | G3 | G4 |
| | | Y-004 cream (0.05%) | 10Y cream (0.05%) | 10Y cream (0.025%) |
| Administration site | Anterior back | Posterior back | Anterior back | Posterior back |

Before the start of the experiment, the marked administration region was shaved for hair removal and skin preparation, ensuring thorough removal without trauma. The shaved area should exceed the marked region. The administration region was marked using a marker pen, cleaned with soapy water and purified water before administration, and wiped dry with a dry gauze.

A quantitative and equal amount of the test article (1 mL/kg) was adhered to the administration site, and then the formulation was evenly spread onto the shaved and marked skin site of the animal using an application tool (for example, a triangular glass rod). After the administration formulation was evenly applied, the site was covered with elastic breathable cotton gauze to ensure that the gauze was fixed at the administration site and the skin surface was breathable. Considering that the animal's movements such as scratching and rubbing could affect the effective coverage of the drug, a mesh elastic bandage was used for fixation to prevent the administration from being affected by the animal's resistive movements.

Six hours after application of the test article, the skin at the administration site was gently washed with soap and purified water to remove residual unabsorbed cream. Skin irritation was observed and recorded during three consecutive days of administration of the corresponding test article in each cycle, in order to compare the short-term irritation differences among the four groups of test articles after three consecutive days of skin administration.

### Clinical observation and skin irritation reaction scoring:

After each administration, observations were performed once at 6 hours (before cleaning and removal of the drug) and at 24 hours (before the next administration), with a focus on observing whether any skin reactions or symptoms such as erythema, scab formation, edema, skin thickening, etc. were present at the administration site. On the last day of each cycle (D3, D6, D11, D16), photographs of the administration sites were taken to record skin reactions. Specifically, the photographs were taken as much as possible within 10 min to 1 hour after the removal of the administration to record skin reactions at the administration site, and skin irritation reaction scoring was performed with reference to the skin irritation reaction scoring criteria in the "Technical Guidelines for Studies on Drug Irritation, Sensitization and Hemolysis".

### Test results:

Under the conditions of this test, after 3 consecutive days of administration, G1 blank cream group and G2 Y-004 cream 0.05% (500 µg/g) specification group showed no skin irritation in miniature Bama pigs (skin irritation intensity score of 0), and no abnormal skin reactions or symptoms were observed. In contrast, G3 10Y cream 0.05% (500 µg/g) specification group and G4 10Y cream 0.025% (250 µg/g) specification group exhibited skin irritation in the miniature Bama pigs, mainly manifested as erythema, redness and swelling, and secondary scab formation and desquamation. The severity and duration of the symptoms were dose-dependent to a certain extent. The highest skin irritation intensity scores for the 250 µg/g and 500 µg/g specifications were 2 and 4, respectively, with certain individual variability. No test article-related abnormal changes were observed in clinical signs, body weight, or food intake.

### Biological test example 3

### Evaluation of the pharmacodynamic effects of compounds on a New Zealand rabbit ear acne model

### Experimental design:

Male New Zealand rabbits qualified for quarantine inspection (Suzhou Xishan Biotechnology Co., Ltd., certificate No. 370823241100331316) were selected for model establishment. For each animal, the left ear was evenly applied with 0.4-0.5 mL/ear of 75% oleic acid using a disinfected cotton swab fully soaked with the 75% oleic acid, while the right ear was left untreated. The application was performed once daily for 11 consecutive days of modeling. On Day 7 of modeling (MDay7), the administration region (2 cm × 3 cm) was determined based on the symptoms of ear acne in the animals, and the administration region was marked using a small animal-specific tattoo machine. On Day 11 of modeling (MDay11), 24 animals with moderate and moderately severe scores from visual observation of the modeled rabbit ears were selected and randomly and evenly divided into: a model control group (Y-004 blank cream, same as the blank cream group in biological test example 2), a tretinoin cream 0.025% control group (commercially available, specification 250 µg/g, active ingredient: tretinoin, containing 250 µg tretinoin per gram of cream), and a Y-004 cream 0.025% group (specification 250 µg/g, active ingredient: the compound Y-004 of the present disclosure, containing 250 µg of the compound Y-004 of the present disclosure per gram of cream, with excipient composition identical to the blank cream of the model control group except for the active ingredient), with 8 animals per group, all male. Approximately 24 hours after the last modeling, the corresponding control/test article was administered within the marked region at 0.06 mL/ear, once daily. At 6 hours ± 0.5 hours after each administration, residual drug was cleaned by washing with a cotton ball soaked in 0.9% sodium chloride injection. Administration was continued for 7 consecutive days, and necropsy was performed 24 hours after the last administration.

During the test, general symptoms of the animals and skin changes at the modeling/administration site were observed and recorded daily; the body weight of the animals was measured once per week; visual observation scoring of the modeling/administration region was performed before the first administration and before necropsy.

### Visual observation grading criteria for acne manifestations

| **Grade** | **Score** | **Criteria for determination** |
|---|---|---|
| Normal | 0 | Normal skin, no acne |
| Mild (Grade I) | 1 | Comedones^{#} as the main skin lesions, possibly with a small number of papules and pustules; the total number of lesions was < 30 |
| Moderate (Grade II) | 2 | Comedones with a moderate number of papules and pustules. The total number of lesions was between 31 and 50. |
| Moderately severe (Grade III) | 3 | Based on moderate comedones, accompanied by a large number of papules and pustules, occasional large inflammatory lesions, widely distributed, with a few nodules formed. The total number of lesions was between 51 and 100. |
| Severe (Grade IV) | 4 | In addition to the above skin lesions, accompanied by more than 3 nodules or cysts |

| | | |
|---|---|---|
| **Note:** # Before necropsy, due to the therapeutic effect or self-recovery of the model control, the comedones during the modeling period have transformed into dilated and elevated follicular orifices. | | |

### Definition of terms:

1. Comedo: A tiny white macule or papule, 0.5-3 mm in diameter, mostly less than 1 mm.
2. Papule: Small, slightly firm, red protrusion with a diameter of less than 0.5 cm.
3. Pustule: A well-defined elevation of the skin appeared at the top of the papule, containing yellowish-white exudate.
4. Nodule/Cyst: Located subcutaneously with no surface skin breakage, mostly formed from comedones that have undergone an inflammatory reaction but have not been completely destroyed, with the inner wall repaired and closed to form a firm foreign body or a cystic cavity.

### Test results:

General condition of animals: During the administration period, the animals in each group were in good general condition, with no obvious abnormalities observed.

Body weight: During the administration period, the body weights of animals in each group were relatively stable, with no obvious abnormalities observed. After 7 consecutive days of administration, the body weights of the animals among the groups were basically consistent (P > 0.05).

Visual observation scores of the administration region: Before the first administration (on the day of grouping, Day 11 of modeling), the visual observation scores of acne manifestations in the administration regions of animals in the model control group (blank cream group), tretinoin cream 0.025% control group, and Y-004 cream 0.025% group were basically consistent (P > 0.05). After 7 consecutive days of administration, the visual observation scores of acne manifestations in the administration regions of animals in tretinoin cream 0.025% control group and Y-004 cream 0.025% group were both lower than those in the model control group (blank cream group) at the same time point, with a more pronounced reduction in the Y-004 cream group (P < 0.05). The visual observation scores of the animals during the test period (Mean ± SD) are shown in FIG. 1 (Note: n=8 for each group. * *P < 0.05* for each group compared with the model control group, *P* > *0.05* for the Y-004 cream group compared with the tretinoin cream control group.)

The Y-004 cream 0.025% group of the present disclosure can significantly reduce the visual observation scores of acne manifestations in the New Zealand rabbit ear acne model, demonstrating an obvious improvement effect.

## Claims

1. A compound of formula I or a pharmaceutically acceptable salt thereof, 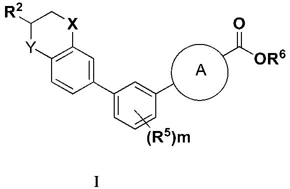
A is 6- to 10-membered arylene or 5- to 6-membered heteroarylene; the 5- to 6-membered heteroarylene has 1, 2, or 3 heteroatoms independently selected from the group consisting of N, O, and S;
Y is -NR¹- or -CR¹R'-;
R¹ is H or C₁₋₆ alkyl;
R' is H or C₁₋₆ alkyl;
R² is H or C₁₋₆ alkyl; or, R¹ and R², together with the intervening atom, form a 3- to 8-membered heterocycle or a C₃₋₈ carbocycle; the 3- to 8-membered heterocycle has 1, 2, or 3 heteroatoms independently selected from the group consisting of N, O, and S; the 3- to 8-membered heterocycle and C₃₋₈ carbocycle are optionally substituted with one or more R²⁻¹; each R²⁻¹ is independently C₁₋₆ alkyl, C₁₋₆ alkoxy, or halogen;
X is -NR³- or -CR³R⁴-;
R³ is H or C₁₋₆ alkyl;
R⁴ is H or C₁₋₆ alkyl;
R⁵ is H, C₁₋₆ alkyl, C₁₋₆ alkyl substituted with one or more R^{5a}, C₁₋₆ alkoxy, or C₁₋₆ alkoxy substituted with one or more R^{5a};
each R^{5a} is independently halogen or hydroxyl;
m is 0, 1, 2, or 3;
R⁶ is H or C₁₋₆ alkyl;
when Y is -CR¹R'-, R¹ and R², together with the intervening atom, form a 3- to 8-membered heterocycle or a C₃₋₈ carbocycle; the 3- to 8-membered heterocycle has 1, 2, or 3 heteroatoms independently selected from the group consisting of N, O, and S.

2. The compound of formula I or the pharmaceutically acceptable salt thereof according to claim 1, wherein the compound of formula I satisfies one or more of the following conditions:
(1) in A, the 6- to 10-membered arylene is phenylene or naphthylene, for example, phenylene;
(2) in A, the 5- to 6-membered heteroarylene has 1 heteroatom, and the heteroatom is preferably N, for example, pyridylene, and for another example, 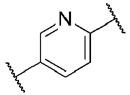 ( 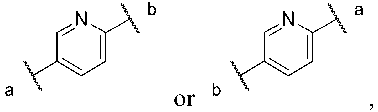 wherein the a end is connected to phenylene, and the b end is connected to carbonyl);
(3) in R¹, the C₁₋₆ alkyl is methyl, ethyl, *n*-propyl, isopropyl, *n*-butyl, isobutyl, *sec*-butyl, or *tert*-butyl, for example, methyl or ethyl;
(4) in R', the C₁₋₆ alkyl is methyl, ethyl, *n*-propyl, isopropyl, *n*-butyl, isobutyl, *sec*-butyl, or *tert*-butyl, for example, methyl or ethyl;
(5) in R², the C₁₋₆ alkyl is methyl, ethyl, *n*-propyl, isopropyl, *n*-butyl, isobutyl, *sec*-butyl, or *tert*-butyl, for example, methyl or ethyl;
(6) in the C₃₋₈ carbocycle formed by R¹ and R² together with the intervening atom, the C₃₋₈ carbocycle is monocyclic, such as C₄₋₆ monocyclic carbocycle, for example, cyclopentane;
(7) in the 3- to 8-membered heterocycle formed by R¹ and R² together with the intervening atom, the 3- to 8-membered heterocycle is monocyclic;
(8) in the 3- to 8-membered heterocycle formed by R¹ and R² together with the intervening atom, the 3- to 8-membered heterocycle is 4- to 6-membered heterocycle, preferably 4- to 6-membered monocyclic heterocycle, for example, 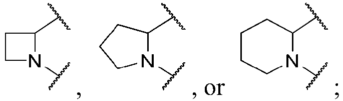
(9) in the 3- to 8-membered heterocycle formed by R¹ and R² together with the intervening atom, the number of heteroatoms is 1, and the heteroatom is preferably N;
(10) in R³, the C₁₋₆ alkyl is methyl, ethyl, *n*-propyl, isopropyl, *n*-butyl, isobutyl, *sec*-butyl, or *tert*-butyl, for example, methyl;
(11) in R⁴, the C₁₋₆ alkyl is methyl, ethyl, *n*-propyl, isopropyl, *n*-butyl, isobutyl, *sec*-butyl, or *tert*-butyl, for example, methyl;
(12) in R⁵, the C₁₋₆ alkyl is methyl, ethyl, *n*-propyl, isopropyl, *n*-butyl, isobutyl, *sec*-butyl, or *tert*-butyl;
(13) in R⁵, the C₁₋₆ alkoxy is methoxy, ethoxy, n-propoxy, isopropoxy, n-butoxy, isobutoxy, sec-butoxy, or *tert*-butoxy, for example, ethoxy, n-propoxy, or n-butoxy;
(14) in R^{5a}, the halogen is fluorine, chlorine, bromine, or iodine;
(15) in R⁵, the C₁₋₆ alkyl substituted with one or more R^{5a} is C₁₋₆ alkyl substituted with one R^{5a}, for example, C₁₋₆ alkyl substituted with one hydroxyl;
(16) in R⁵, the C₁₋₆ alkoxy substituted with one or more R^{5a} is C₁₋₆ alkoxy substituted with one R^{5a}, for example, C₁₋₆ alkoxy substituted with one hydroxyl, and for another example, 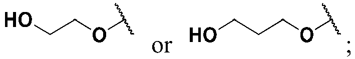
(17) in R⁶, the C₁₋₆ alkyl is methyl, ethyl, *n*-propyl, isopropyl, *n*-butyl, isobutyl, *sec*-butyl, or *tert*-butyl;
(18) in R²⁻¹, the C₁₋₆ alkyl is methyl, ethyl, *n*-propyl, isopropyl, *n*-butyl, isobutyl, *sec*-butyl, or *tert*-butyl;
(19) in R²⁻¹, the C₁₋₆ alkoxy is methoxy, ethoxy, *n*-propoxy, isopropoxy, *n*-butoxy, isobutoxy, *sec-*butoxy, or *tert*-butoxy;
(20) in R²⁻¹, the halogen is fluorine, chlorine, bromine, or iodine;
(21) the more than one refers to 2 or 3.

3. The compound of formula I or the pharmaceutically acceptable salt thereof according to claim 1 or 2, wherein the compound of formula I satisfies one or more of the following conditions:
(1) m is 0, 1, or 2, preferably, m is 1;
(2) A is 6- to 10-membered arylene or 5- to 6-membered heteroarylene; the 5- to 6-membered heteroarylene has 1 heteroatom, and the heteroatom is N; preferably, A is 6- to 10-membered arylene; more preferably, A is phenylene;
(3) Y is -NR¹- or -CHR¹-; when Y is -CHR¹-, R¹ and R², together with the intervening atom, form a 3- to 8-membered heterocycle or a C₃₋₈ carbocycle; the 3- to 8-membered heterocycle has 1, 2, or 3 heteroatoms independently selected from the group consisting of N, O, and S; preferably, when Y is -CHR¹-, R¹ and R², together with the intervening atom, form a 3- to 8-membered heterocycle or a C₃₋₈ carbocycle; the 3- to 8-membered heterocycle is monocyclic and has 1 heteroatom, and the heteroatom is N; the C₃₋₈ carbocycle is monocyclic (for example, the C₄₋₆ monocyclic carbocycle); more preferably, when Y is -CHR¹-, R¹ and R², together with the intervening atom, form a C₃₋₈ carbocycle; the C₃₋₈ carbocycle is monocyclic;
(4) Y is -NR¹-;
(5) R¹ is H or C₁₋₆ alkyl; R² is H or C₁₋₆ alkyl; or, R¹ and R², together with the intervening atom, form a 3- to 8-membered heterocycle or a C₃₋₈ carbocycle; the 3- to 8-membered heterocycle has 1, 2, or 3 heteroatoms independently selected from the group consisting of N, O, and S; preferably, R¹ is H or C₁₋₆ alkyl; R² is H or C₁₋₆ alkyl; or, R¹ and R², together with the intervening atom, form a 3- to 8-membered heterocycle (for example, a 4- to 6-membered heterocycle) or a C₃₋₈ carbocycle (for example, a C₄₋₆ carbocycle); the 3- to 8-membered heterocycle is monocyclic and has 1 heteroatom, and the heteroatom is N; the C₃₋₈ carbocycle is monocyclic; more preferably, R¹ is C₁₋₆ alkyl; R² is H or C₁₋₆ alkyl; or, R¹ and R², together with the intervening atom, form a 3- to 8-membered heterocycle (for example, a 4- to 6-membered heterocycle) or a C₃₋₈ carbocycle (for example, a C₄₋₆ carbocycle); the 3- to 8-membered heterocycle is monocyclic and has 1 heteroatom, and the heteroatom is N; the C₃₋₈ carbocycle is monocyclic;
(6) X is -CR³R⁴-;
(7) R³ is C₁₋₆ alkyl;
(8) R⁴ is C₁₋₆ alkyl;
(9) R⁵ is C₁₋₆ alkoxy or C₁₋₆ alkoxy substituted with one or more R^{5a}, preferably, R⁵ is C₁₋₆ alkoxy substituted with one R^{5a};
(10) R^{5a} is fluorine, chlorine, bromine, or hydroxyl, preferably, R^{5a} is hydroxyl;
(11) R⁶ is H, methyl, ethyl, *n*-propyl, isopropyl, *n*-butyl, isobutyl, *sec*-butyl, or *tert*-butyl, preferably, R⁶ is H;
(12) Y is -NR¹-, R¹ and R², together with the intervening atom, form a 3- to 8-membered heterocycle; the 3- to 8-membered heterocycle has 1, 2, or 3 heteroatoms independently selected from the group consisting of N, O, and S, wherein at least one of the heteroatoms is N; the 3- to 8-membered heterocycle is optionally substituted with one or more R²⁻¹; each R²⁻¹ is independently C₁₋₆ alkyl, C₁₋₆ alkoxy, or halogen; preferably, when Y is -NR¹-, R¹ and R², together with the intervening atom, form a 3- to 8-membered heterocycle; the 3- to 8-membered heterocycle is monocyclic and has 1 heteroatom, and the heteroatom is N; the 3- to 8-membered heterocycle is optionally substituted with one or more R²⁻¹; each R²⁻¹ is independently C₁₋₆ alkyl, C₁₋₆ alkoxy, or halogen.

4. The compound of formula I or the pharmaceutically acceptable salt thereof according to any one of claims 1-3, wherein the compound of formula I satisfies one or more of the following conditions:
(1) 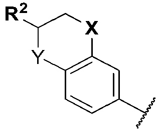 is 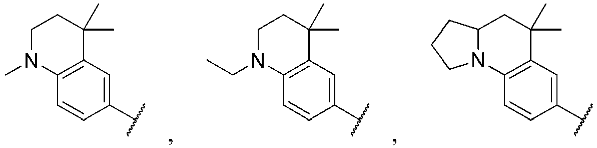 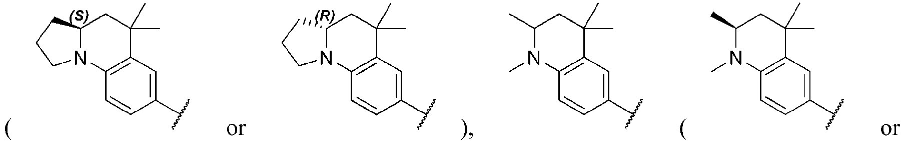 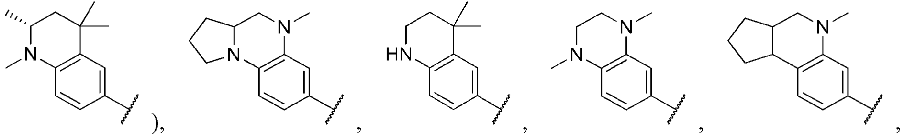 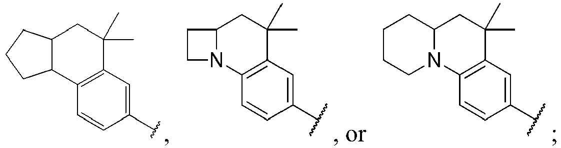
(2) R⁶ is hydrogen;
(3) R⁵ is 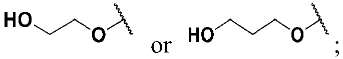
(4) 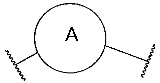 is 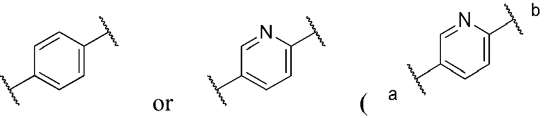 or 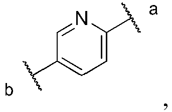 wherein the a end is connected to phenylene, and the b end is connected to carbonyl).

5. The compound of formula I or the pharmaceutically acceptable salt thereof according to any one of claims 1-4, wherein the compound of formula I is Scheme 1, Scheme 2, Scheme 3, or Scheme 4:
Scheme 1:
m is 0, 1, or 2;
A is 6- to 10-membered arylene or 5- to 6-membered heteroarylene; the 5- to 6-membered heteroarylene has 1 heteroatom, and the heteroatom is N;
Y is -NR¹- or -CHR¹-;
R¹ is H or C₁₋₆ alkyl; R² is H or C₁₋₆ alkyl; or, R¹ and R², together with the intervening atom, form a 3- to 8-membered heterocycle (for example, a 4- to 6-membered heterocycle) or a C₃₋₈ carbocycle (for example, a C₄₋₆ carbocycle); the 3- to 8-membered heterocycle is monocyclic and has 1 heteroatom, and the heteroatom is N; the C₃₋₈ carbocycle is monocyclic;
X is -NR³- or -CR³R⁴-;
R³ is H or C₁₋₆ alkyl;
R⁴ is H or C₁₋₆ alkyl;
R⁵ is C₁₋₆ alkoxy or C₁₋₆ alkoxy substituted with one or more R^{5a}.
R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl;
when Y is -CHR¹-, R¹ and R², together with the intervening atom, form a C₃₋₈ carbocycle; the C₃₋₈ carbocycle is monocyclic (for example, C₄₋₆ monocyclic carbocycle);
Scheme 2:
m is 0, 1, or 2;
A is 6- to 10-membered arylene;
Y is -NR¹-;
R¹ is H or C₁₋₆ alkyl; R² is H or C₁₋₆ alkyl; or, R¹ and R², together with the intervening atom, form a 3- to 8-membered heterocycle (for example, a 4- to 6-membered heterocycle); the 3- to 8-membered heterocycle is monocyclic and has 1 heteroatom, and the heteroatom is N;
X is -CR³R⁴-;
R³ is H or C₁₋₆ alkyl;
R⁴ is H or C₁₋₆ alkyl;
R⁵ is C₁₋₆ alkoxy or C₁₋₆ alkoxy substituted with one or more R^{5a};
R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl;
Scheme 3:
m is 1;
A is 6- to 10-membered arylene or 5- to 6-membered heteroarylene; the 5- to 6-membered heteroarylene has 1 heteroatom, and the heteroatom is N;
Y is -NR¹- or -CHR¹-;
R¹ is H or C₁₋₆ alkyl; R² is H or C₁₋₆ alkyl; or, R¹ and R², together with the intervening atom, form a 3- to 8-membered heterocycle (for example, a 4- to 6-membered heterocycle) or a C₃₋₈ carbocycle (for example, a C₄₋₆ carbocycle); the 3- to 8-membered heterocycle is monocyclic and has 1 heteroatom, and the heteroatom is N; the C₃₋₈ carbocycle is monocyclic;
X is -NR³- or -CR³R⁴-; R³ is C₁₋₆ alkyl; R⁴ is C₁₋₆ alkyl;
R⁵ is C₁₋₆ alkoxy substituted with one R^{5a};
R^{5a} is hydroxyl;
R⁶ is H;
when Y is -CHR¹-, R¹ and R², together with the intervening atom, form a C₃₋₈ carbocycle (for example, a C₄₋₆ carbocycle), and the C₃₋₈ carbocycle is monocyclic;
Scheme 4:
m is 1;
A is 6- to 10-membered arylene;
Y is -NR¹-;
R¹ is C₁₋₆ alkyl; R² is H or C₁₋₆ alkyl; or, R¹ and R², together with the intervening atom, form a 3- to 8-membered heterocycle (for example, a 4- to 6-membered heterocycle); the 3- to 8-membered heterocycle is monocyclic and has 1 heteroatom, and the heteroatom is N;
X is -CR³R⁴-; R³ is C₁₋₆ alkyl; R⁴ is C₁₋₆ alkyl;
R⁵ is C₁₋₆ alkoxy substituted with one R^{5a};
R^{5a} is hydroxyl;
R⁶ is H.

6. The compound of formula I or the pharmaceutically acceptable salt thereof according to any one of claims 1-4, wherein the compound of formula I is a compound of formula I-1 or a compound of formula I-2: 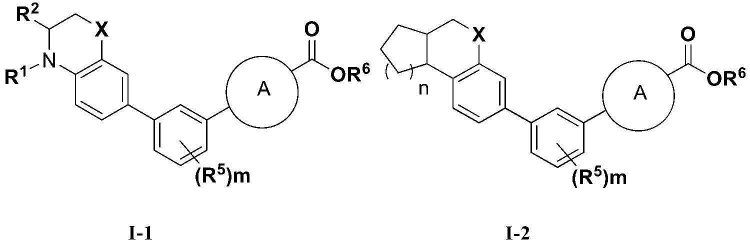 (for example, 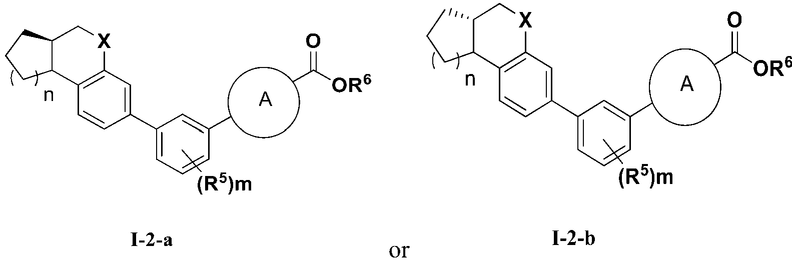 );
n is 0, 1, 2, or 3; m, A, R¹, R², X, R⁵, and R⁶ are as defined in any one of claims 1-4;
preferably, the compound of formula I satisfies one or two of the following conditions:
(1) in the compound of formula I-1,
m is 0, 1, or 2;
A is 6- to 10-membered arylene;
R¹ is H or C₁₋₆ alkyl; R² is H or C₁₋₆ alkyl; or, R¹ and R², together with the intervening atom, form a 3- to 8-membered heterocycle (for example, a 4- to 6-membered heterocycle); the 3- to 8-membered heterocycle is monocyclic and has 1 heteroatom, and the heteroatom is N;
X is -CR³R⁴-; R³ is H or C₁₋₆ alkyl; R⁴ is H or C₁₋₆ alkyl;
R⁵ is C₁₋₆ alkoxy or C₁₋₆ alkoxy substituted with one or more R^{5a};
R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl; preferably, in the compound of formula I-1,
m is 1; A is phenylene;
R¹ is C₁₋₆ alkyl; R² is H or C₁₋₆ alkyl; or, R¹ and R², together with the intervening atom, form a 3- to 8-membered heterocycle (for example, a 4- to 6-membered heterocycle); the 3- to 8-membered heterocycle is monocyclic and has 1 heteroatom, and the heteroatom is N;
X is -CR³R⁴-; R³ is C₁₋₆ alkyl; R⁴ is C₁₋₆ alkyl;
R⁵ is C₁₋₆ alkoxy substituted with one R^{5a}.
R^{5a} is hydroxyl;
R⁶ is H;
(2) in the compound of formula I-2 (for example, compound of I-2-a or I-2-b),
n is 0, 1, or 2;
m is 0, 1, or 2;
A is 6- to 10-membered arylene;
X is -NR³- or -CR³R⁴-; R³ is H or C₁₋₆ alkyl; R⁴ is H or C₁₋₆ alkyl;
R⁵ is C₁₋₆ alkoxy or C₁₋₆ alkoxy substituted with one or more R^{5a}.
R^{5a} is fluorine, chlorine, bromine, or hydroxyl;
R⁶ is H, methyl, ethyl, n-propyl, isopropyl, n-butyl, isobutyl, sec-butyl, or *tert-butyl;*
preferably, in the compound of formula I-2 (for example, compound of I-2-a or I-2-b), n is 1; m is 1; A is phenylene;
X is -CR³R⁴-; R³ is C₁₋₆ alkyl; R⁴ is C₁₋₆ alkyl;
R⁵ is C₁₋₆ alkoxy substituted with one R^{5a}.
R^{5a} is hydroxyl;
R⁶ is H;
more preferably, the compound of formula I is a compound of formula I-1-a, a compound of formula I-1-b, or a compound of formula I-1-c: 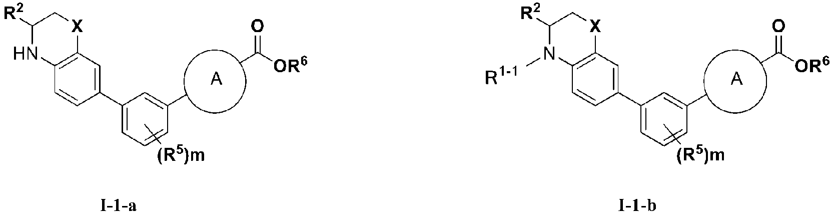 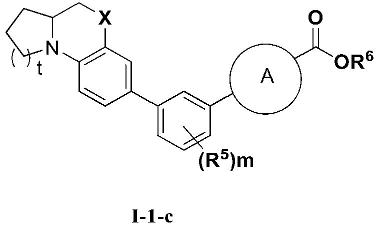 (for example, 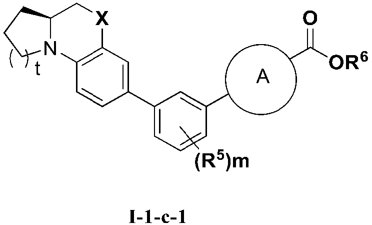 or 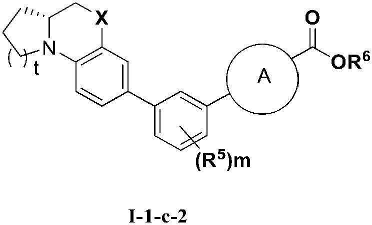
t is 0, 1, 2, or 3; R¹⁻¹ is C₁₋₆ alkyl; m, A, R², X, R⁵, and R⁶ are as defined in any one of claims 1-4; in R¹⁻¹, the C₁₋₆ alkyl is preferably methyl, ethyl, n-propyl, isopropyl, n-butyl, isobutyl, *sec*-butyl, or *tert*-butyl, for example, methyl or ethyl;
further preferably, the compound of formula I satisfies one or more of the following conditions:
(1) in the compound of formula I-1-a,
m is 0, 1, or 2;
A is 6- to 10-membered arylene;
R² is H or C₁₋₆ alkyl;
X is -CR³R⁴-; R³ is H or C₁₋₆ alkyl; R⁴ is H or C₁₋₆ alkyl;
R⁵ is C₁₋₆ alkoxy or C₁₋₆ alkoxy substituted with one or more R^{5a};
R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl;
preferably, in the compound of formula I-1-a,
m is 1; A is phenylene;
R² is H or C₁₋₆ alkyl;
X is -CR³R⁴-; R³ is C₁₋₆ alkyl; R⁴ is C₁₋₆ alkyl;
R⁵ is C₁₋₆ alkoxy substituted with one R^{5a};
R^{5a} is hydroxyl;
R⁶ is H;
(2) in the compound of formula I-1-b,
m is 0, 1, or 2;
A is 6- to 10-membered arylene;
R¹⁻¹ is C₁₋₆ alkyl;
R² is H or C₁₋₆ alkyl;
X is -CR³R⁴-; R³ is H or C₁₋₆ alkyl; R⁴ is H or C₁₋₆ alkyl;
R⁵ is C₁₋₆ alkoxy or C₁₋₆ alkoxy substituted with one or more R^{5a};
R^{5a} is fluorine, chlorine, bromine, or hydroxyl;
R⁶ is H, methyl, ethyl, *n*-propyl, isopropyl, *n*-butyl, isobutyl, *sec-*butyl, or *tert*-butyl;
preferably, in the compound of formula I-1-b,
m is 1; A is phenylene;
R¹⁻¹ is C₁₋₆ alkyl;
R² is H or C₁₋₆ alkyl;
X is -CR³R⁴-; R³ is C₁₋₆ alkyl; R⁴ is C₁₋₆ alkyl;
R⁵ is C₁₋₆ alkoxy substituted with one R^{5a};
R^{5a} is hydroxyl;
R⁶ is H;
(3) in the compound of formula I-1-c (for example, compound of I-1-c-1 or I-1-c-2),
t is 0, 1, or 2;
m is 0, 1, or 2;
A is 6- to 10-membered arylene;
X is -CR³R⁴-; R³ is H or C₁₋₆ alkyl; R⁴ is H or C₁₋₆ alkyl;
R⁵ is C₁₋₆ alkoxy or C₁₋₆ alkoxy substituted with one or more R^{5a};
R^{5a} is fluorine, chlorine, bromine, or hydroxyl;
R⁶ is H, methyl, ethyl, *n*-propyl, isopropyl, *n*-butyl, isobutyl, *sec-*butyl, or *tert*-butyl;
preferably, in the compound of formula I-1-c (for example, compound of I-1-c-1 or I-1-c-2),
t is 1 or 2; m is 0 or 1; A is phenylene;
X is -CR³R⁴-; R³ is C₁₋₆ alkyl; R⁴ is C₁₋₆ alkyl;
R⁵ is C₁₋₆ alkoxy substituted with one or more R^{5a}.
R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl;
more preferably, in the compound of formula I-1-c (for example, compound of I-1-c-1 or I-1-c-2),
t is 1; m is 1; A is phenylene;
X is -CR³R⁴-; R³ is C₁₋₆ alkyl; R⁴ is C₁₋₆ alkyl;
R⁵ is C₁₋₆ alkoxy substituted with one R^{5a};
R^{5a} is hydroxyl;
R⁶ is H.

7. The compound of formula I or the pharmaceutically acceptable salt thereof according to any one of claims 1-4, wherein the compound of formula I is a compound of formula I-1-1 or a compound of formula I-2-1: 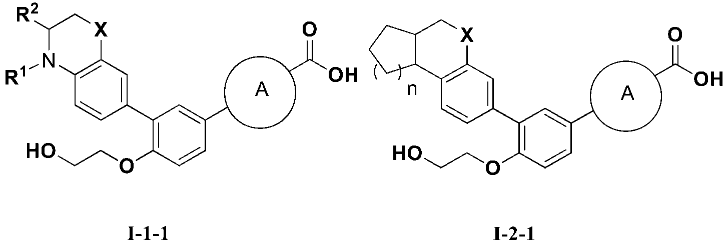 (for example, 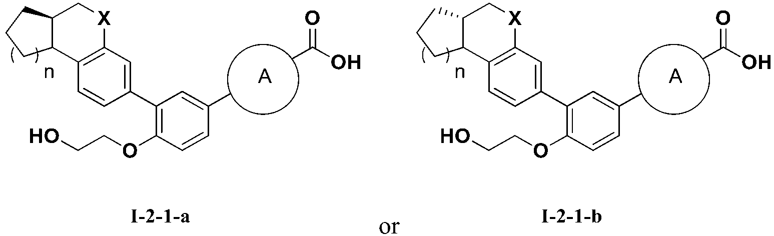 )
n is 0, 1, 2, or 3; A, R¹, R², and X are as defined in any one of claims 1 to 4;
preferably, the compound of formula I satisfies one or two of the following conditions:
(1) in the compound of formula I-1-1,
A is 6- to 10-membered arylene;
R¹ is H or C₁₋₆ alkyl; R² is H or C₁₋₆ alkyl; or, R¹ and R², together with the intervening atom, form a 3- to 8-membered heterocycle (for example, a 4- to 6-membered heterocycle); the 3- to 8-membered heterocycle is monocyclic and has 1 heteroatom, and the heteroatom is N;
X is -CR³R⁴-; R³ is H or C₁₋₆ alkyl; R⁴ is H or C₁₋₆ alkyl;
preferably, in the compound of formula I-1-1,
A is phenylene;
R¹ is C₁₋₆ alkyl; R² is H or C₁₋₆ alkyl; or, R¹ and R², together with the intervening atom, form a 3- to 8-membered heterocycle; the 3- to 8-membered heterocycle is monocyclic and has 1 heteroatom, and the heteroatom is N;
X is -CR³R⁴-; R³ is C₁₋₆ alkyl; R⁴ is C₁₋₆ alkyl;
(2) in the compound of formula I-2-1 (for example, I-2-1-a, I-2-1-b),
n is 0, 1, or 2; A is 6- to 10-membered arylene;
X is -CR³R⁴-; R³ is H or C₁₋₆ alkyl; R⁴ is H or C₁₋₆ alkyl;
preferably, in the compound of formula I-2-1 (for example, I-2-1-a, I-2-1-b),
n is 1; A is phenylene;
X is -CR³R⁴-; R³ is C₁₋₆ alkyl; R⁴ is C₁₋₆ alkyl;
more preferably, the compound of formula I is a compound of formula I-1-1-1, a compound of formula I-1-1-2, or a compound of formula 1-1-1-3: 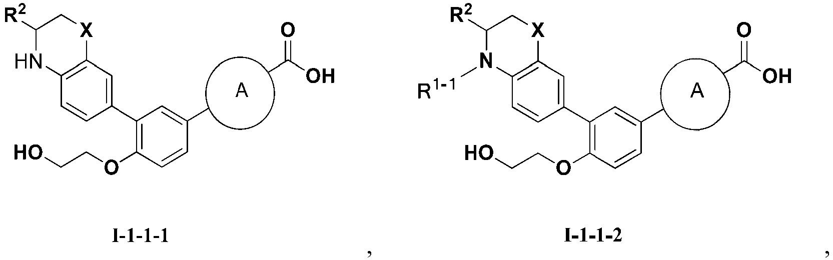 or 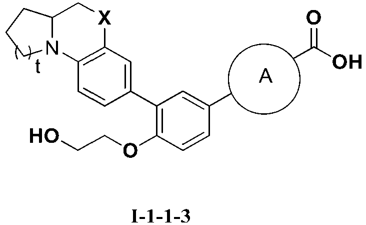 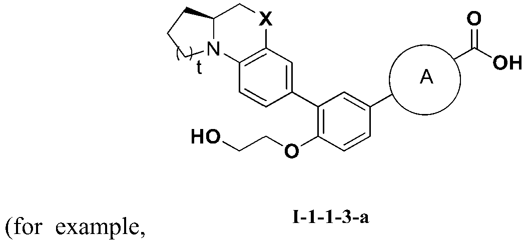 or 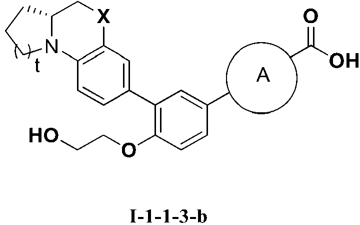 );
t is 0, 1, 2, or 3; R¹⁻¹ is C₁₋₆ alkyl; A, R², and X are as defined in any one of claims 1 to 4; in R¹⁻¹, the C₁₋₆ alkyl is preferably methyl, ethyl, *n*-propyl, isopropyl, *n*-butyl, isobutyl, *sec-*butyl, or *tert*-butyl, for example, methyl or ethyl;
further preferably, the compound of formula I satisfies one or more of the following conditions:
(1) in the compound of formula I-1-1-1,
A is 6- to 10-membered arylene; R² is H or C₁₋₆ alkyl; X is -CR³R⁴-; R³ is H or C₁₋₆ alkyl; R⁴ is H or C₁₋₆ alkyl;
preferably, in the compound of formula I-1-1-1,
A is phenylene; R² is H or C₁₋₆ alkyl; X is -CR³R⁴-; R³ is C₁₋₆ alkyl; R⁴ is C₁₋₆ alkyl;
(2) in the compound of formula I-1-1-2,
A is 6- to 10-membered arylene; R¹⁻¹ is C₁₋₆ alkyl; R² is H or C₁₋₆ alkyl; X is -CR³R⁴-; R³ is H or C₁₋₆ alkyl; R⁴ is H or C₁₋₆ alkyl;
preferably, in the compound of formula I-1-1-2,
A is phenylene; R¹⁻¹ is C₁₋₆ alkyl; R² is H or C₁₋₆ alkyl; X is -CR³R⁴-; R³ is C₁₋₆ alkyl; R⁴ is C₁₋₆ alkyl;
(3) in the compound of formula I-1-1-3 (for example, I-1-1-3-a, I-1-1-3-b),
t is 0, 1, or 2; A is a 6- to 10-membered arylene;
X is -CR³R⁴-; R³ is H or C₁₋₆ alkyl; R⁴ is H or C₁₋₆ alkyl;
preferably, in the compound of formula I-1-1-3 (for example, I-1-1-3-a, I-1-1-3-b),
t is 1 or 2; A is phenylene; X is -CR³R⁴-; R³ is C₁₋₆ alkyl; R⁴ is C₁₋₆ alkyl;
more preferably, in the compound of formula I-1-1-3 (for example, I-1-1-3-a, I-1-1-3-b),
t is 1; A is phenylene; X is -CR³R⁴-; R³ is C₁₋₆ alkyl; R⁴ is C₁₋₆ alkyl.

8. The compound of formula I or the pharmaceutically acceptable salt thereof according to any one of claims 1-4, wherein the compound of formula I may be a compound of formula I-A, a compound of formula I-B, a compound of formula I-C, or a compound of formula I-D 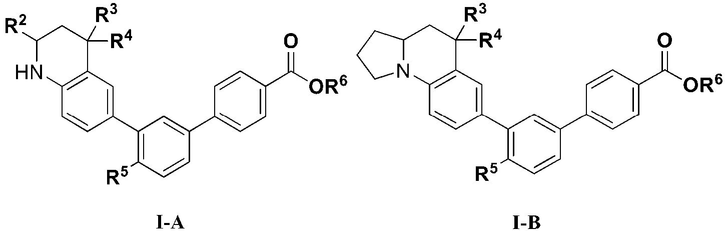 (for example, 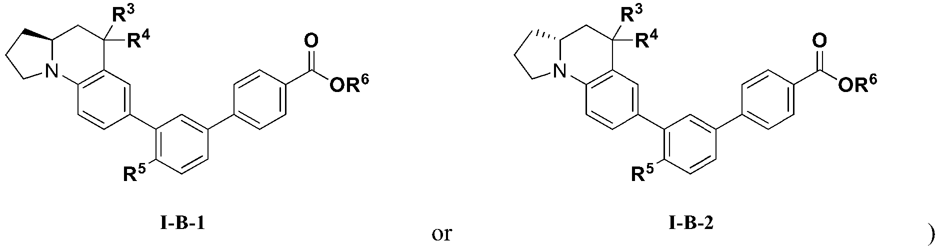 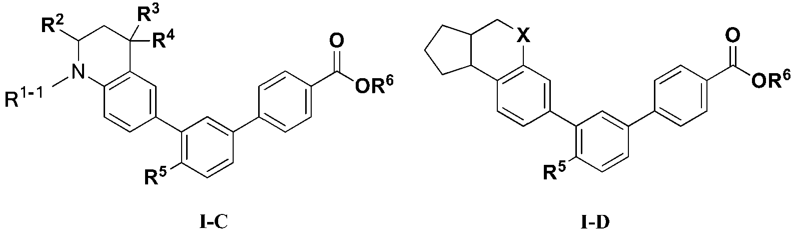 (for example, 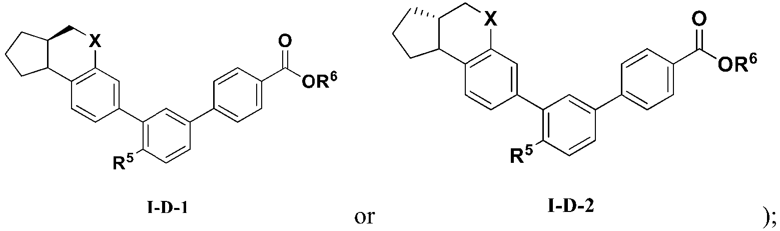
R¹⁻¹ is C₁₋₆ alkyl; X, R², R³, R⁴, R⁵, and R⁶ are as defined in any one of claims 1 to 4; in R¹⁻¹, the C₁₋₆ alkyl is preferably methyl, ethyl, *n*-propyl, isopropyl, *n*-butyl, isobutyl, *sec*-butyl, or *tert*-butyl, for example, methyl or ethyl;
preferably, the compound of formula I satisfies one or more of the following conditions:
(1) in the compound of formula I-A,
R² is H or C₁₋₆ alkyl; R³ is H or C₁₋₆ alkyl; R⁴ is H or C₁₋₆ alkyl;
R⁵ is C₁₋₆ alkoxy or C₁₋₆ alkoxy substituted with one or more R^{5a}; R^{5a} is fluorine, chlorine, bromine, or hydroxyl;
R⁶ is H, methyl, ethyl, *n*-propyl, isopropyl, *n*-butyl, isobutyl, *sec-*butyl, or *tert*-butyl;
preferably, in the compound of formula I-A,
R² is H or C₁₋₆ alkyl; R³ is C₁₋₆ alkyl; R⁴ is C₁₋₆ alkyl;
R⁵ is C₁₋₆ alkoxy substituted with one R^{5a}; R^{5a} is hydroxyl; R⁶ is H;
(2) in the compound of formula I-B (for example, I-B-1, I-B-2),
R³ is H or C₁₋₆ alkyl; R⁴ is H or C₁₋₆ alkyl;
R⁵ is C₁₋₆ alkoxy or C₁₋₆ alkoxy substituted with one or more R^{5a}; R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl;
preferably, in the compound of formula I-B (for example, I-B-1, I-B-2),
R³ is C₁₋₆ alkyl; R⁴ is C₁₋₆ alkyl;
R⁵ is C₁₋₆ alkoxy substituted with one R^{5a}; R^{5a} is hydroxyl; R⁶ is H;
(3) in the compound of formula I-C,
R¹⁻¹ is C₁₋₆ alkyl;
R² is H or C₁₋₆ alkyl; R³ is H or C₁₋₆ alkyl; R⁴ is H or C₁₋₆ alkyl;
R⁵ is C₁₋₆ alkoxy or C₁₋₆ alkoxy substituted with one or more R^{5a}; R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl;
preferably, in the compound of formula I-C,
R¹⁻¹ is C₁₋₆ alkyl;
R² is H or C₁₋₆ alkyl; R³ is C₁₋₆ alkyl; R⁴ is C₁₋₆ alkyl;
R⁵ is C₁₋₆ alkoxy substituted with one R^{5a}; R^{5a} is hydroxyl; R⁶ is H;
(4) in the compound of formula I-D (for example, I-D-1, I-D-2),
X is -NR³- or -CR³R⁴-; R³ is H or C₁₋₆ alkyl; R⁴ is H or C₁₋₆ alkyl;
R⁵ is C₁₋₆ alkoxy or C₁₋₆ alkoxy substituted with one or more R^{5a}; R^{5a} is fluorine, chlorine, bromine, or hydroxyl;
R⁶ is H, methyl, ethyl, *n*-propyl, isopropyl, *n*-butyl, isobutyl, *sec*-butyl, or *tert*-butyl;
preferably, in the compound of formula I-D (for example, I-D-1, I-D-2),
X is -CR³R⁴-; R³ is C₁₋₆ alkyl; R⁴ is C₁₋₆ alkyl; R⁵ is C₁₋₆ alkoxy substituted with one R^{5a}; R^{5a} is hydroxyl;
R⁶ is H.

9. The compound of formula I or the pharmaceutically acceptable salt thereof according to claim 1, wherein the compound of formula I is any one of the following compounds:

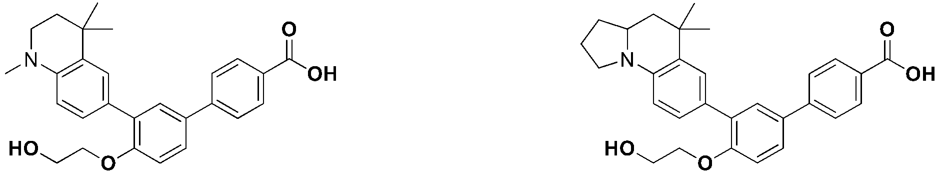

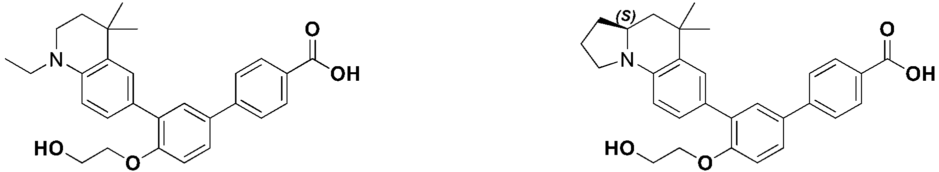

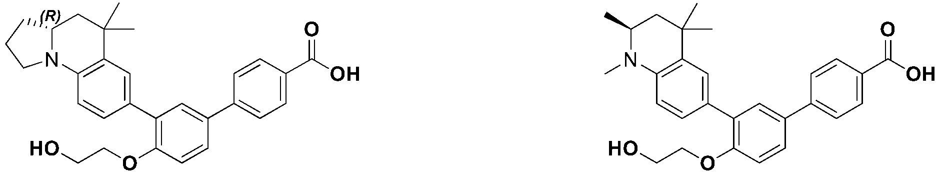

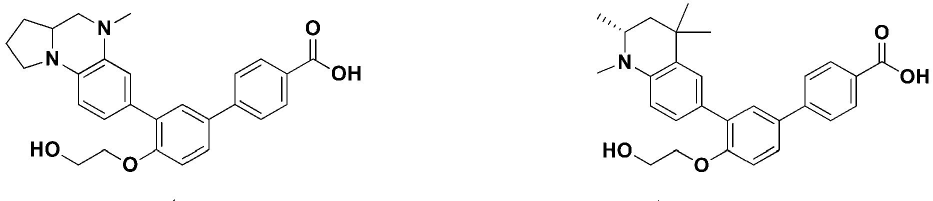

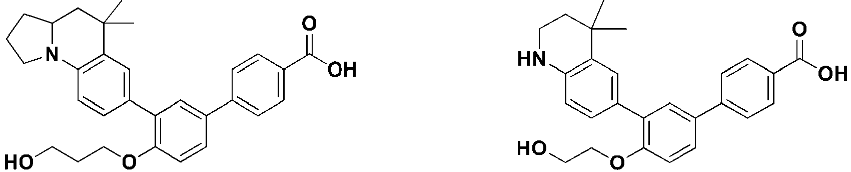

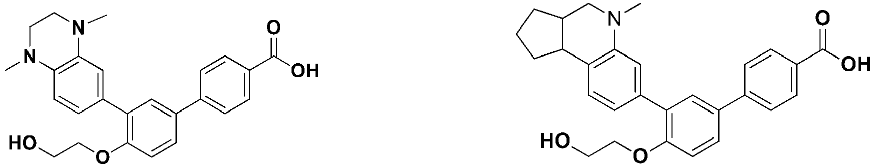

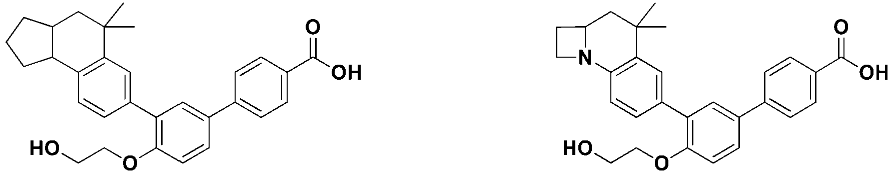

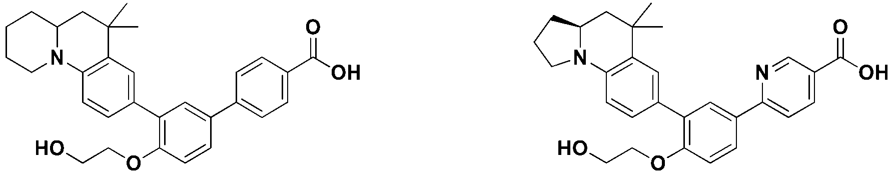

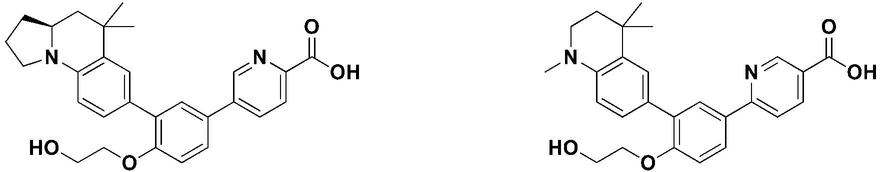

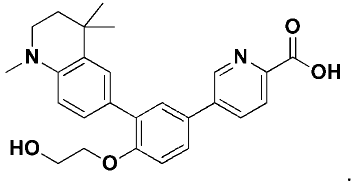

10. A pharmaceutical composition comprising (i) the compound of formula I or the pharmaceutically acceptable salt thereof as defined in any one of claims 1 to 9; and (ii) a pharmaceutically acceptable carrier.

11. Use of the compound of formula I or the pharmaceutically acceptable salt thereof as defined in any one of claims 1 to 9, or the pharmaceutical composition as defined in claim 10, as a retinoic acid receptor γ agonist.

12. Use of the compound of formula I or the pharmaceutically acceptable salt thereof as defined in any one of claims 1 to 9, or the pharmaceutical composition as defined in claim 10, in the manufacture of a medicament for treating and/or preventing a disease associated with retinoic acid receptor γ;
preferably, the disease associated with retinoic acid receptor γ may be acne (including acne vulgaris, rosacea, nodulocystic acne, acne conglobata, and secondary acne caused by sunlight or medication), comedo, psoriasis, ichthyosis, cutaneous keratinization disorder, pigmentation, or dry eye syndrome.

13. Use of the compound of formula I or the pharmaceutically acceptable salt thereof as defined in any one of claims 1 to 9, or the pharmaceutical composition as defined in claim 10, in the manufacture of a medicament;
preferably, the medicament is used for treating and/or preventing acne (including acne vulgaris, rosacea, nodulocystic acne, acne conglobata, and secondary acne caused by sunlight or medication), comedo, psoriasis, ichthyosis, cutaneous keratinization disorder, pigmentation, or dry eye syndrome.

14. A compound of formula II or a salt thereof 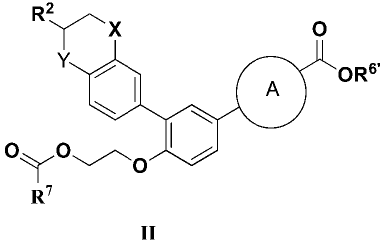
R^{6'} is C₁₋₆ alkyl; R⁷ is C₁₋₆ alkyl; A, R², X, and Y are as defined in any one of claims 1 to 9;
in R⁷, the C₁₋₆ alkyl is preferably methyl, ethyl, *n*-propyl, isopropyl, *n*-butyl, isobutyl, *sec-*butyl, or *tert*-butyl, for example, methyl;
in R^{6'}, the C₁₋₆ alkyl is preferably methyl, ethyl, *n*-propyl, isopropyl, *n*-butyl, isobutyl, sec-butyl, or *tert*-butyl, for example, ethyl;
preferably, the compound of formula II is any one of the following compounds:

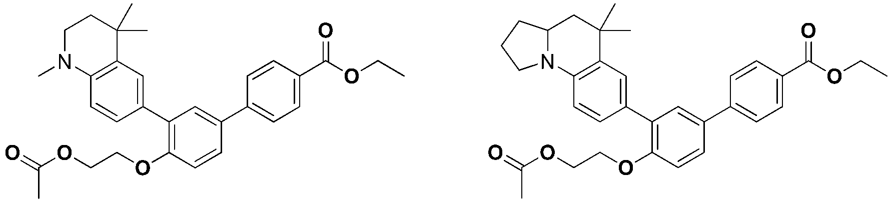

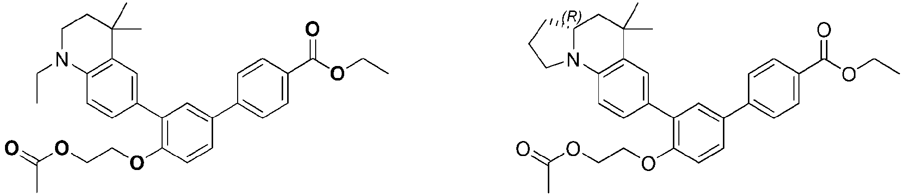

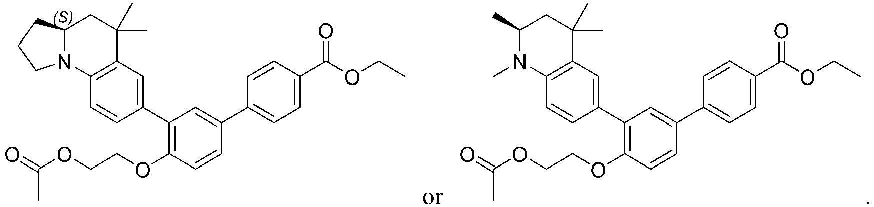

15. A method for preparing a compound of formula I, comprising the following step: subjecting a compound of formula II to a hydrolysis reaction as shown below (for example, at 50-70°C, further for example, at 60°C) in an organic solvent (for example, an alcohol solvent, further for example, methanol) in the presence of a base (for example, an alkali metal hydroxide, further for example, sodium hydroxide, still further for example, 1 mol/L aqueous sodium hydroxide solution) to obtain the compound of formula I; 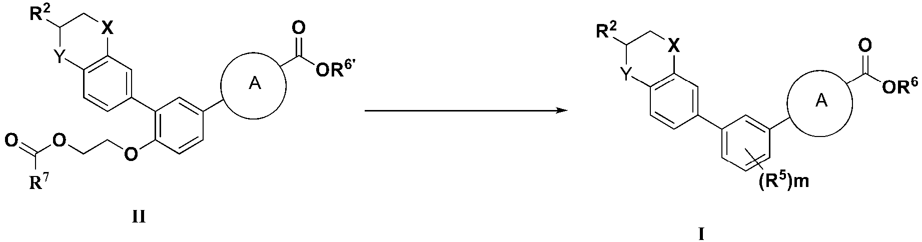
R⁶ is H; R⁵ is 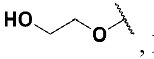 R⁵ is attached at the para position of A, m is 1; A, X, Y, and R² are as defined in any one of claims 1 to 9, and R⁶ and R⁷ are as defined in claim 14;
preferably, the method for preparing the compound of formula I further comprises the following step: subjecting a compound of formula III and a compound of formula IV to a coupling reaction as shown in the following scheme (for example, at 100-120°C, further for example, at 110°C) in a solvent (for example, a mixed solvent of water and a cyclic ether solvent, further for example, a mixed solvent of water and dioxane, still further for example, dioxane and water in a volume ratio of 20:3 or 4:1) in the presence of a catalyst (for example, a palladium catalyst, further for example, Pd(dppf)Cl₂) and an alkali metal halide salt (for example, an alkali metal fluoride salt, further for example, KF) to obtain the compound of formula II; 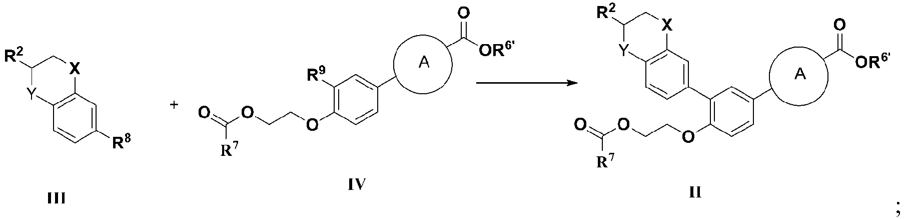
R⁸ is a boronate ester group 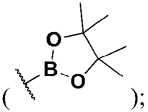 R⁹ is halogen (for example, bromine); A, X, Y, and R² are as defined in any one of claims 1 to 9; R^{6'} and R⁷ are as defined in claim 14.

16. A method for preparing a compound of formula II, comprising the following step: subjecting a compound of formula III and a compound of formula IV to a coupling reaction as shown in the following scheme (for example, at 100-120°C, further for example, at 110°C) in a solvent (for example, a mixed solvent of water and a cyclic ether solvent, further for example, a mixed solvent of water and dioxane, still further for example, dioxane and water in a volume ratio of 20:3 or 4:1) in the presence of a catalyst (for example, a palladium catalyst, further for example, Pd(dppf)Cl₂) and an alkali metal halide salt (for example, an alkali metal fluoride salt, further for example, KF) to obtain the compound of formula II; 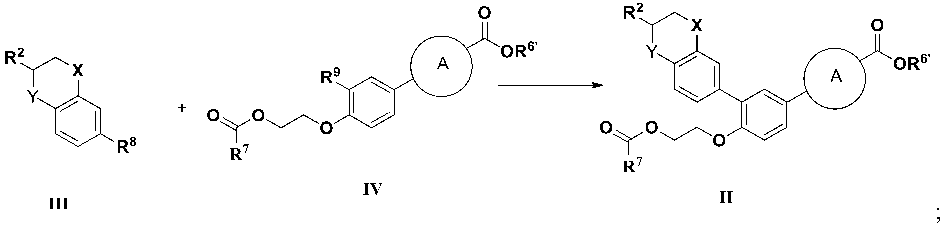 R⁸ is a boronate ester group 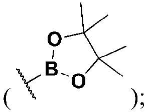 R⁹ is halogen (for example, bromine); A, X, Y, and R² are as defined in any one of claims 1 to 9; R^{6'} and R⁷ are as defined in claim 14.
